(19) Europäisches Patentamt / European Patent Office / Office européen des brevets

(11) **EP 4 289 972 A1**

(12) **EUROPEAN PATENT APPLICATION**

(43) Date of publication:
**13.12.2023 Bulletin 2023/50**

(21) Application number: **23178173.3**

(22) Date of filing: **08.06.2023**

(51) International Patent Classification (IPC):
***C12Q 1/689*** (2018.01)

(52) Cooperative Patent Classification (CPC):
(C-Sets available)
**C12Q 1/689** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.06.2022 EP 22178076**

(71) Applicant: **Congen Biotechnologie GmbH
13125 Berlin (DE)**

(72) Inventor: **Mergemeier, Steffen
10243 Berlin (DE)**

(74) Representative: **Hertin und Partner
Rechts- und Patentanwälte PartG mbB
Kurfürstendamm 54/55
10707 Berlin (DE)**

Remarks:
The complete document including Reference Table(s) and the Sequence Listing(s) can be downloaded from the EPO website

(54) **METHOD FOR DETECTING METHICILLIN RESISTANT STAPHYLOCOCCUS AUREUS**

(57)    The invention relates to a method to detect a methicillin-resistant *Staphylococcus aureus* (MRSA) strain or genetic material thereof in a sample by a nucleic acid amplification reaction, by detecting target sequences of a staphylococcal cassette chromosome mec (SC-Cmec). Related oligonucleotides, primers, probes, oligonucleotide sets and kits are also provided.

**EP 4 289 972 A1**

**(Cont. next page)**

(52) Cooperative Patent Classification (CPC): (Cont.)

C-Sets
**C12Q 1/689, C12Q 2600/156**

**Description**

The invention is in the field of molecular detection and/or diagnostics, in particular detection of microorganisms using nucleic acid detection techniques.

The invention relates to a method to detect a methicillin-resistant *Staphylococcus aureus* (MRSA) strain or genetic material thereof in a sample by a nucleic acid amplification reaction, by detecting target sequences of a staphylococcal cassette chromosome mec (SCCmec). In embodiments, the target sequences to be detected have not been described previously in the context of MRSA detection and represent a unique and novel genetic element of a SCCmec cassette found in some MRSA strains. As described in more detail below, the invention is based on identifying this unique and novel genetic element and employing detection of said element in reducing or avoiding false negative results as would be generated using existing MRSA detection productions, kits and methods.

The invention relates to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises: a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and a second sequence region (10) comprising a sequence of a methicillin-resistant *Staphylococcus aureus* (MRSA) and a mecA gene (17), adjacent to said first region (8), thereby forming a boundary between said first and second regions (11), the method comprising: contacting a sample with a first primer (12) and a second primer (14), of which at least one hybridizes with the second region (10), capable of generating a first amplicon (15) that comprises a sequence of the second region (10), and detecting said first amplicon (15), wherein detecting said amplicon (15) indicates the presence of MRSA or genetic material thereof in the sample.

In other aspects the invention relates to a kit for carrying out the method of the invention. In other aspects the invention relates to a set of oligonucleotides, preferably configured to be suitable for carrying out the method of the invention.

## BACKGROUND OF THE INVENTION

*Staphylococcus aureus* (SA) has long been known as a human pathogen causing a wide spectrum of diseases. Since its first identification over 50 years ago, methicillin-resistant *Staphylococcus aureus* (MRSA) has become one of the world's most significant pathogens, often causing severe infections in hospitalized patients, which can be particularly fatal in immunocompromised or post-surgical patients. *Staphylococcus aureus* is one of the most common causes of sepsis, skin and soft tissue infections, and pneumonia.

*Staphylococcus aureus* occurs as part of normal skin flora in healthy humans and is mostly found in the nasopharynx or on the skin. *Staphylococcus aureus* has the ability to adapt to rapidly changing environments, for example by developing diverse resistance mechanisms to antimicrobial agents such as penicillin and other commonly used antibiotics. *Staphylococcus aureus* strains also resistant to methicillin emerged shortly after its use in medical practice, with these strains also showing resistances to penicillin, streptomycin and tetracycline.

The pathogenic potential of methicillin resistant *Staphylococcus aureus* (MRSA) as a clinical pathogen typically lies in SA strains comprising a methicillin-type antibiotic resistance gene (mecA).

Methicillin resistance in MRSA is conferred by a low affinity beta-lactam-resistant penicillin-binding protein (PBP), which was acquired from coagulase-negative staphylococci (CNS) and is encoded by the chromosomal mecA gene. The MRSA specific PBP functions as a beta-lactam resistant transpeptidase. The mecA gene is absent in methicillin-susceptible or "methicillin-sensitive" SA (MSSA) but is commonly expressed in other *Staphylococcus* species and is highly conserved.

The mecA gene is comprised within a genetic element called staphylococcal cassette chromosome mec (SCCmec), which is inserted in the MRSA chromosome. SCCmec is a mobile genetic element characterized by the presence of terminal inverted and direct repeats, a set of site-specific recombinase genes (ccrA and ccrB), and the mecA gene complex.

Hiramatsu et al. revealed that SCCmec is commonly integrated at the specific site in the chromosome of MSSA at the 3' end of a novel open reading frame (ORF), orfX. Today, different methods for the detection and identification of MRSA have been described. Hiramatsu et al. (US 6,156,507) and Huletsky et al. (WO 2007/044873 A2), for example, developed a MRSA-specific PCR assay, which employs primers that specifically hybridize to the right ends of three different SCCmec sequences in combination with primers specific for the SA chromosome and corresponding to the nucleotide sequence on the right side of the SCCmec integration site.

Since the description of the MRSA-specific junction between the SCCmec sequence and the orfX cassette of the SA genome (termed the mec right extremity junction (MREJ)) by Hiramatsu, numerous diagnostic methods have been developed that are able to detect a variety of MRSA strains, based on their specific MREJ sequences, such as WO 2002/099034 and WO 2009/085221. In addition, PCR detection systems for MRSA have been described in the art, such as in EP 1529847 or EP 2807273, which employ a primer that hybridizes across the junction between the SSCmec cassette and the orfX cassette of the SA genome.

However, the inventors recently discovered that standard MRSA detection methods that analyze the boundary described by Hiramatsu ("Hiramatsu boundary", as described in US 6,156,507) between orfX and MRSA-specific sequences are unable to detect a newly identified type of MRSA. As described in more detail below, the newly discovered MRSA comprise between the resistance conferring MRSA/mecA sequence and the orfX cassette an additional sequence found in (associated with) known MSSA strains. As a result, the "Hiramatsu boundary" in these strains reveals an orfX sequence positioned adjacent to a sequence found in MSSA strains. Hence, the standard analysis of said orfX boundary using the PCR primers and methods according to Hiramatsu and the related prior art, would not detect this newly identified type of MRSA, thus generating false-negative results.

In view of the relevance of MRSA infections for the individual and public health, difficulties in providing reliable detection methods, and the frequent detection of novel MRSA sequence variants, novel and reliable methods to detect new MRSA strains are urgently needed.

## SUMMARY OF THE INVENTION

In light of the prior art the technical problem underlying the present invention is to provide means for detecting methicillin resistant *Staphylococcus aureus* (MRSA) strains that overcome the disadvantages of those methods known in the prior art. A further object of the invention is seen as providing means for detecting MRSA that reduce or avoid false-negative results potentially generated by the methods of the prior art. A further object of the invention is seen as providing means for detecting new MRSA strains not previously identified.

This problem is solved by the features of the independent claims. Preferred embodiments of the present invention are provided by the dependent claims.

In one aspect the invention relates to a method to detect a methicillin-resistant *Staphylococcus aureus* (MRSA) strain or genetic material thereof in a sample by a nucleic acid amplification reaction, by detecting target sequences of a staphylococcal cassette chromosome mec (SCCmec).

In embodiments, the target sequences to be detected have not been described previously in the context of MRSA detection.

In embodiments, the target sequences to be detected represent a unique and novel genetic element of a SCCmec cassette found in newly discovered MRSA strains.

As described in more detail below, the invention is based on identifying a unique and novel genetic element, or unique combinations of known genetic elements, not previously described in the context of MRSA or MRSA detection, and employing methods, kits, oligonucleotides and/or other reagents for the detection of said elements, thus preferably reducing or avoiding false negative results as would be generated using existing MRSA detection productions, kits and methods.

*Aspects and embodiments related to the detection of MRSA in which MSSA sequence is adjacent to orfX:*

In one aspect, the invention therefore relates to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and
- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:

contacting a sample with a first primer (12) and a second primer (14), of which at least one hybridizes with the second region (10), capable of generating a first amplicon (15) that comprises a sequence of the second region (10),

and detecting said first amplicon (15), wherein detecting said amplicon (15) indicates the presence of MRSA or genetic material thereof in the sample.

The surprising and beneficial effect of the method according to the present invention is the possibility to also detect MRSA strains, which do comprise a methicillin resistance-conferring sequence (mecA) in an MRSA SCCmec sequence, although this MRSA SCCmec sequence is not positioned directly adjacent to the orfX sequence/element of the SA genome, but in in a second sequence region, which is separated from the orfX region by a first sequence region, which is known from an MSSA strain.

In embodiments, first sequence region may be referred to as associated with, derived from or corresponding to a MSSA strain.

The detection of said novel MRSA strains is facilitated by the present method in a reliable and efficient manner and overcomes the shortcoming of the methods of the prior art, which are unable to detect said MRSA strains and would deliver false-negative results in case of their presence.

At the time of the present invention, methods for detecting the specific MRSA strains comprising a first and a second sequence region, as described herein, have not been described in the prior art and no efforts have been directed towards detecting said strains.

The inventors have identified new genetic elements of these MRSA strains, which comprise a nucleic acid sequence that is associated with MSSA strains (a first sequence region), which is located in between the SCCmec region conferring the methicillin resistance and comprising the mecA gene (a second sequence region), and the usually analyzed "Hiramatsu" orfX boundary.

Hence, using the PCR primers and method proposed by Hiramatsu, and any related prior art method, such MRSA strains cannot be detected. PCR using these methods, employing primers positioned across the "Hiramatsu" boundary, would not amplify a PCR product, as these primers are designed not to hybridize to and thus not to amplify MSSA sequences. Consequently, in case of infections with said newly described MRSA strains, the standard MRSA detection methods would deliver false-negative results, which might entail fatal consequences for an affected patient.

In other words, as none of the prior art methods is able to detect MRSA strains that comprise a (first) region associated with a MSSA strain positioned between the orfX sequence and the SCCmec region conferring methicillin resistance (second region), there exists an urgent need for means that facilitate the identification of said new MRSA strains or variants.

The present invention solves this need, as it will be evident from the present disclosure and the examples provided herein.

In embodiments, the method of the present invention reliably enables detection of MRSA strains that have a "Hiramatsu boundary" between SCCmec and orfX which resembles an MSSA strain. Thus, the orfX-first region boundary resembles MSSA, i.e. the first region sequence indicates the presence of MSSA. These strains however comprise another novel boundary, within the SCCmec cassette, between the first region and second region. The first region (MSSA sequence) thus lies between the orfX and second regions. The second region comprises SCCmec cassette sequence that resembles MRSA and also comprises a mecA gene. Using the methods and other reagents of the present invention, nucleic acid amplification is carried out of the second region, or across the boundary between the first and second regions, thus identifying MRSA strains with this novel genetic element, which are not detected using common means available in the art.

In embodiments, MRSA strains can be detected, that comprise a segment of MSSA sequence (first sequence region (8)) that is positioned between an orfX sequence (1) and a mecA sequence (positioned in the second sequence region (10)). In embodiments, the boundary between said first and second regions (11) may be positioned directly next to a mecA gene, which is present in the second sequence region. In other embodiments, the mecA gene may be positioned in the second region (10) but distant to the boundary (11). The exact position of the mecA gene is therefore not determinative to the method.

In embodiments, the presence of a mecA gene in the MRSA to be detected can assessed using an amplification reaction with primers and optionally probes that hybridize to mecA. Such primers are known in the art and/or can be developed without undue effort.

Upon identification of said novel MRSA strains, which comprise a new type of SCCmec sequence (comprising the first and second sequence region) adjacent to an orfX sequence, the inventors developed the method described herein for detecting said specific novel MRSA strains.

Said novel MRSA strains comprise a new type of SCCmec sequence (16) that comprises a first sequence region (or "first region") comprising a sequence of a methicillin-sensitive *Staphylococcus aureus* (MSSA) (8), which is positioned adjacent to a chromosomal orfX sequence (1) and forms a boundary (9) between the first sequence region and the orfX sequence. The new type of SCCmec sequence further comprises a second sequence region (10; or "second region") that comprises a nucleic acid sequence of (or associated with) a methicillin-resistant *Staphylococcus aureus* (MRSA) and comprises a mecA gene (17), adjacent to said first region, and thus forms a boundary (11) between said first and second sequence regions.

Aspects of the invention relate to a method comprising contacting a sample with a first and a second primer, of which at least one hybridizes or binds to the second region, and which is capable of generating a first amplicon that comprises a sequence of the second region (the MRSA associated region), and optionally also a sequence of the first sequence region (8; the MSSA associated region) and their boundary (11)) and wherein the detection of said first amplicon enables the identification of the presence of said novel MRSA or genetic material thereof in a sample.

In a preferred embodiment the invention relates to a method, wherein the first primer (12) hybridizes with the first region (8), and the second primer (14) hybridizes with the second region (10), wherein the method comprises generating a first amplicon (15) comprising sequences of the first (8) and second regions (10), and the first amplicon (15) comprises the boundary (11) between said first (8) and second regions (10).

In a preferred embodiment, the first sequence region (8) is at least 1000 nt in length, preferably 2000 nt, more preferably 3000 nt in length.

In embodiments, the first sequence region (8) comprises between 30 and 3000 consecutive nucleotides or base pairs (bp), for example, at least 30, 40, 50, 60, 70, 80, 90 100, 125, 150, 175, 200, 225, 250, 275, 300, 325, 350, 375, 400, 425, 450, 475, 500, 525, 550, 575, 800, 825, 850, 875, 900, 925, 950, 975, 1000, 1250, 1500, 1750, 2000, 2250, 2500, 2750, 3000 or more nucleotides or base pairs. In embodiments, the first sequence region (8) comprises 500 to 15000 consecutive nucleotides or base pairs (bp), such as 1000, 2000, 3000, 4000, 5000, 6000, 7000, 8000, 9000, 10000, 11000, 12000, 13000, 14000 or 15000 bp.

In embodiments, the second sequence region (10) comprises between 30 and 3000 consecutive nucleotides or base pairs (bp), for example, at least 30, 40, 50, 60, 70, 80, 90 100, 125, 150, 175, 200, 225, 250, 275, 300, 325, 350, 375, 400, 425, 450, 475, 500, 525, 550, 575, 800, 825, 850, 875, 900, 925, 950, 975, 1000, 1250, 1500, 1750, 2000, 2250, 2500, 2750, 3000, or more nucleotides or base pairs.

In embodiments, the first sequence region (8) comprises at least 1000 bp of sequence typically found in MSSA strains adjacent to the orfX sequence (1).

*Embodiments relating to additional detection of the "Hiramatsu boundary":*

The present method not only enables the identification of the newly described MRSA strains that comprise MSSA sequence (8) adjacent to the orfX cassette (1), in between the MRSA sequence (10) and the orfX cassette (1), but also of MRSA strains that comprise a MRSA sequence (2) directly adjacent to the orfX cassette (1).

In embodiments of the invention, the present method comprises additionally:

contacting the sample with a third primer (3) that hybridizes with the orfX sequence (1), and a fourth primer (5) that hybridizes with an adjacent sequence region of an MRSA comprising SCCmec sequence (2), capable of generating a second amplicon (6) comprising SCCmec (2) and orfX (1) sequences and a boundary (7) between said SCCmec (2) and orfX (1) sequences, and

detecting the first or second amplicons.

The inventors have identified further MRSA strains that comprise novel sequences adjacent to the boundary (7) to the orfX sequence. Due to said novel sequences, next to the boundary (7) to the orfX cassette, prior art primers would not be able to bind to said MRSA sequences and would therefore fail to identify or detect said MRSA strains in a sample.

Surprisingly, after identifying such MRSA sequence variants, the inventors have developed a new set of primers that enable the amplification and detection of MRSA strains in a simple, cost effective and straightforward PCR assay.

As prior art primers would not be able to reliably bind all newly identified MRSA sequence variations (2), adjacent to the orfX cassette, a nucleic acid sequence analysis, e.g. next generation sequencing, would be required to identify the presence of said yet unknown MRSA variants. NGS analysis would pose a high financial burden and require an extended processing time, wherefore it would not be suitable for standard clinical practice. Otherwise, standard PCR assays would simply deliver false-negative results.

In the context of the present method, one of the herein described third or fourth primers binds/hybridizes to the genetic material of said novel MRSA strains on the side of the orfX cassette of the boundary (7) (the boundary between the orfX cassette (1) and the MRSA sequence(2)), while the other primer binds/hybridizes to the MRSA sequence (2) on the other side of the boundary (7). If such a novel MRSA strain is present in the sample, a nucleic amplification reaction with the third and fourth primer according to the invention generates an amplicon (6), which can be detected, for example, with a probe (4) that hybridizes with said amplicon. The present method therefore surprisingly enables the detection of MRSA strains or variants that have been newly identified by the inventors and that could not be reliably detected using prior art methods.

Embodiments of the invention therefore relate to an MRSA-identification method that employs a set of primers that hybridize (or bind to) a DNA region upstream and to a region downstream of the junction or "boundary" (7) between the MRSA sequence (2) and the SA genome (orfX cassette; (1)) facilitating the amplification and generation of an amplicon spanning the junction or boundary (7) between the MRSA sequence (2) and the orfX cassette of the SA genome (1). The junction or boundary (7) between orfX and MRSA SCCmec sequence may also be referred to as the "Hiramatsu boundary" or "Hiramatsu junction".

The combination of a set of first and second primers according to the invention with a set of third and fourth primers according to the invention results in an advantageous effect, namely that any MRSA strain may be detected, if present in a sample, independently of whether the MRSA sequence is located directly adjacent to the orfX sequence, or if the MRSA sequence and the orfX sequence are separated by a MSSA sequence. In both cases of MRSA infection, one of the two primer sets (either the first and second primer or the third and fourth primer) would lead to the detection of the MRSA. In summary, the present method in combination with the herein described primers enables a reliable identification and detection of newly discovered MRSA strains, which could not be detected with prior art methods.

In one embodiment, detecting the first amplicon (15) from the first (12) and second primers (14) according to the invention and not detecting the second amplicon (6) from the third (3) and fourth primers (5) indicates the presence of MRSA or genetic material thereof in the sample.

The above embodiment relates to sample where a MRSA as described herein is present, that comprises MSSA sequence (8) adjacent to the orfX cassette (1), in between the MRSA sequence (10) and the orfX cassette (1).

In one embodiment, detecting the first or second amplicons, wherein _not_ detecting the first amplicon (15) from the first (12) and second primers (14) of claim 1 and detecting the second amplicon (6) from the third (3) and fourth primers (5) indicates the presence of MRSA or genetic material thereof in the sample.

The above embodiment relates to sample where a MRSA as described herein is present, that comprises a MRSA sequence (2) directly adjacent to the orfX cassette (1).

In one embodiment, detecting the **first** amplicon (15) from the **first** (12) and **second primers** (14) of claim 1, or detecting the second amplicon (6) from the third (3) and fourth primers (5), indicates the presence of MRSA or genetic material thereof in the sample.

Either positive amplification result may therefore represent detection of MRSA.

In embodiments, either the first amplicon (15) or the second amplicon (6) is detected, to determine the presence of MRSA, wherein both first (15) and second (6) amplicons cannot be detected.

Thus, in one embodiment, the method comprises additionally:

contacting the sample with a third primer (3) that hybridizes with the orfX sequence (1), and a fourth primer (5) that hybridizes with an adjacent sequence region of an MRSA comprising SCCmec sequence (2), capable of generating a second amplicon (6) comprising SCCmec (2) and orfX (1) sequences and a boundary (7) between said SCCmec (2) and orfX (1) sequences, and

detecting the first and/or second amplicons,

wherein detecting the first amplicon (15) from the first (12) and second primers (14) according to the invention and not detecting the second amplicon (6) from the third (3) and fourth primers (5) indicates the presence of MRSA or genetic material thereof in the sample.

In embodiments, the method is configured to determine both the first (15) and the second amplicon (6) after amplification. In embodiments, a positive MRSA determination is evident when either the first (15) or second amplicon (6) is detected.

In another embodiment of the invention the method comprises additionally:

contacting the sample with a third primer (3) that hybridizes with the orfX sequence (1), and a fourth primer (5) that hybridizes with an adjacent sequence region of an MRSA comprising SCCmec sequence (2), capable of generating a second amplicon (6) comprising SCCmec (2) and orfX (1) sequences and a boundary (7) between said SCCmec (2) and orfX (1) sequences,

detecting the first and/or second amplicons,

wherein _not_ detecting the first amplicon (15) from the first (12) and second primers (14) of claim 1 and detecting the second amplicon (6) from the third (3) and fourth primers (5) indicates the presence of MRSA or genetic material thereof in the sample.

*Embodiments related to the third and fourth primers:*

The following embodiments of the invention provide further information on the target sequences to which the primers of the method hybridize (bind to) and the preferred primer sequences.

In embodiments of the method according to the invention the third primer (3) hybridizes with an orfX sequence (1) according to SEQ ID NO SEQ ID NO 39, 40, or 41, or a sequence of at least 80% sequence identity thereto.

In the above embodiment, the third primer (3) hybridizes with an orfX sequence (1) according to SEQ ID NO SEQ ID NO 39, 40, or 41, or a sequence of at least 90%, or 95%, sequence identity thereto.

In embodiments of the method according to the invention the fourth primer (5) hybridizes with an MRSA SCCmec sequence (2), according to SEQ ID NO 42, 43 or 44, or a sequence of at least 80% sequence identity thereto.

In embodiments of the method according to the invention the fourth primer (5) hybridizes with an MRSA SCCmec sequence (2), according to SEQ ID NO 42, 43 or 44, or a sequence of at least 90%, or 95%, sequence identity thereto.

In embodiments of the method according to the invention the third primer (3) comprises or consists of a sequence according to SEQ ID NO 35 or 36, or a sequence of at least 80% sequence identity thereto.

In embodiments of the method according to the invention the third primer (3) comprises or consists of a sequence according to SEQ ID NO 35 or 36, or a sequence of at least 90%, or 95%, sequence identity thereto.

In embodiments of the method according to the invention the fourth primer (5) comprises or consists of a sequence according to one of SEQ ID NO 19-34, or a sequence of at least 80% sequence identity thereto.

In embodiments of the method according to the invention the fourth primer (5) comprises or consists of a sequence according to one of SEQ ID NO 19-34, or a sequence of at least 90%, or 95%, sequence identity thereto.

Any reference herein to SEQ ID NO 19-34 relates to any one or more of SEQ ID NO 19, 20, 21, 22, 23, 24, 25, 26, 27, 28, 29, 30, 31, 32, 33 and/or 34.

Hence, embodiments of the invention relate to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and

- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:

contacting a sample with a first primer (12) and a second primer (14), of which at least one hybridizes with the second region (10), capable of generating a first amplicon (15) that comprises a sequence of the second region (10), and

contacting the sample with a third primer (3) that hybridizes with the orfX sequence (1), and a fourth primer (5) that hybridizes with an adjacent sequence region of an MRSA comprising SCCmec sequence (2), capable of generating a second amplicon (6) comprising SCCmec (2) and orfX (1) sequences and a boundary (7) between said SCCmec (2) and orfX (1) sequences,

wherein the third primer (3) comprises or consists of a sequence according to SEQ ID NO 35 or 36, or a sequence of at least 80%, 90% or 95% sequence identity thereto, and/or the fourth primer (5) comprises or consists of a sequence according to one of SEQ ID NO 19-34, or a sequence of at least 80%, 90% or 95% sequence identity thereto,

and detecting the first and/or second amplicons,

wherein detecting the first amplicon (15) from the first (12) and second primers (14) of claim 1, or detecting the second amplicon (6) from the third (3) and fourth primers (5), indicates the presence of MRSA or genetic material thereof in the sample.

*Embodiments relating to differentiating between MRSA and MSSA:*

Further, in embodiments the method according to the invention comprises differentiating between (i) methicillin-resistant *Staphylococcus aureus* (MRSA) and (ii) methicillin-sensitive *Staphylococcus aureus* (MSSA) in the sample, wherein detecting the first amplicon (15) from the first (12) and second primers (14) according to the invention and *not* detecting the second amplicon (6) from the third (3) and fourth primers (5) according to the invention indicates the presence of MRSA or genetic material thereof and absence of MSSA in the sample.

In another embodiment said method comprises differentiating between (i) methicillin-resistant Staphylococcus aureus (MRSA) and (ii) methicillin-sensitive *Staphylococcus aureus* (MSSA) in the sample, wherein *not* detecting the first amplicon (15) from the first (12) and second primers (14) of claim 1 and detecting the second amplicon (6) from the third (3) and fourth primers (5) indicates the presence of MRSA or genetic material thereof in the sample and absence of MSSA in the sample.

Analysing a sample that comprises the herein described novel MRSA or genetic material thereof with MRSA-specific primers of the prior art would lead to false-negative results, as these primers are unable to bind to and detect the herein described novel MRSA strains. In addition, some prior art assays use primers specific for MSSA strains, which bind adjacent to the boundary to the orfX cassette first described by Hiramatsu, to distinguish between MRSA and MSSA in a sample. Unfortunately, in cases of the presence of the herein described new MRSA strains that harbour a MSSA sequence in between the MRSA sequence and the orfX cassette, these assays would also lead to false-negative results. They would indicate the presence of a MSSA sequence adjacent to the orfX cassette, without detecting the presence of the MRSA-specific sequence next to said MSSA sequence. Consequently, in these scenarios prior art assays would lead to the wrong assumption that no MRSA is present in a patient sample, which could incur fatal consequences for a patient.

*Further embodiments:*

In one embodiment the method of the present invention is characterized in that the SA strain to be identified is selected from the group consisting of type I, II, III, V, IV a, IV b, IV c, VII, VIII, IX and/or X. In another embodiment the method of the present invention is capable of detecting MRSA of type I, II, III, V, IV a, IV b, IV c, VII, VIII, IX and/orX, especially types I, II, III, V, IV a, IV b and/or IV c. Other MRSA types capable of being detected are XI, XII, XIII, XIV, XV, XVI, XVII, XVIII, XIX or XX. The nomenclature of this embodiment relates to those strains or MRSA for example characterized in WO2007044873, which is hereby incorporated by reference.

The present method enables the identification of different MRSA variants, although each sample to be tested may only contain one particular MRSA strain.

It is a surprising and advantageous aspect of the present invention that different embodiments of the present method enable detection of various MRSA strains in a reliable and efficient manner. In a preferred embodiment of the invention multiple primers can be used in a multiplex manner. Accordingly, in embodiments the method according to the invention comprises a multiplex-nucleic acid amplification and detection reaction, such as a multiplex PCR or multiplex qPCR / real-time PCR. The various primers and/or probes of the present invention bay be combined in any given combination for multiplex amplification, combining all primers and corresponding probes in one reaction or any given sub-group of primers and/or corresponding probes in a number of multiplex reactions, as may be carried out by a skilled person.

In embodiments the MRSA strain to be detected comprises a chromosomal *Staphylococcus* mec (SSCmec) cassette (16), which carries a mecA gene (17), or mecA-equivalent gene.

The presence of a MRSA in a sample can be detected in embodiments of the invention using PCR, real-time or qPCR that identifies the presence of specific nucleic acid sequences using a probe (a specific nucleic acid fragment or molecule) that hybridizes to said specific nucleic acid sequence (target).

*Embodiments related to the sequences of the first and second sequence regions:*

In embodiments of the method according to the invention detecting an amplicon (15, 6) comprises detecting the presence of a signal from an oligonucleotide probe (4, 13) that hybridizes to the sequence of the amplicon (15, 6).

In embodiments of the method according to the invention the first (12) and/or second primers (14), and/or the probe (13) for detecting the first amplicon (15), hybridize to a second sequence region (10) that is present in MRSA and absent in MSSA.

In embodiments of the method according to the invention the second sequence region (10) comprises a sequence of at least the first 30 nucleotides of a sequence according to SEQ ID NO 1, 2 or 3, or a sequence of at least 80% sequence identity thereto, wherein the first nucleotide of the second sequence region (10) is adjacent to the 3' end of the first sequence region (8).

Accordingly, in embodiments the invention relates to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and
- a second sequence region (10) comprising a sequence of a methicillin-resistant *Staphylococcus aureus* (MRSA) and a mecA gene (17), adjacent to said first region (8), thereby forming a boundary between said first and second regions (11), wherein the second sequence region (10) comprises a sequence of at least the first 30 nucleotides of a sequence according to SEQ ID NO 1, 2 or 3, or a sequence of at least 80% sequence identity thereto, wherein the first nucleotide of the second sequence region (10) is adjacent to the 3' end of the first sequence region (8).

the method comprising:
contacting a sample with a first primer (12) and a second primer (14), of which at least one hybridizes with the second region (10), capable of generating a first amplicon (15) that comprises a sequence of the second region (10), and detecting said first amplicon (15), wherein detecting said amplicon (15) indicates the presence of MRSA or genetic material thereof in the sample.

In embodiments of the method according to the invention the first sequence region (8) comprises a sequence of at least the last 30 nucleotides of a sequence according to SEQ ID NO 4, 5 or 6, or a sequence of at least 80% sequence identity thereto, wherein the last nucleotide of the first sequence region (8) is adjacent to the 5' end of the second sequence region (10).

In embodiments of the method according to the invention the first sequence region (8) comprises a sequence of at least the last 30 nucleotides of a sequence according to SEQ ID NO 4, 5 or 6, or a sequence of at least 90%, or 95%, sequence identity thereto, wherein the last nucleotide of the first sequence region (8) is adjacent to the 5' end of the second sequence region (10).

Accordingly, in embodiments the invention relates to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), wherein the first sequence region (8) comprises a sequence of at least the last 30 nucleotides of a sequence according to SEQ ID NO 4, 5 or 6, or a sequence of at least 80% sequence identity thereto, wherein the last nucleotide of the first sequence region (8) is adjacent to the 5' end of the second sequence region (10), and

- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:
contacting a sample with a first primer (12) and a second primer (14), of which at least one hybridizes with the second region (10), capable of generating a first amplicon (15) that comprises a sequence of the second region (10), and detecting said first amplicon (15), wherein detecting said amplicon (15) indicates the presence of MRSA or genetic material thereof in the sample.

In another embodiment of the method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), wherein the first sequence region (8) comprises a sequence of at least the last 30 nucleotides of a sequence according to SEQ ID NO 4, 5 or 6, or a sequence of at least 80% sequence identity thereto, wherein the last nucleotide of the first sequence region (8) is adjacent to the 5' end of the second sequence region (10), and

- a second sequence region (10) comprising a sequence of a methicillin-resistant *Staphylococcus aureus* (MRSA) and a mecA gene (17), adjacent to said first region (8), thereby forming a boundary between said first and second regions (11), wherein
the second sequence region (10) comprises a sequence of at least the first 30 nucleotides of a sequence according to SEQ ID NO 1, 2 or 3, or a sequence of at least 80% sequence identity thereto, wherein the first nucleotide of the second sequence region (10) is adjacent to the 3' end of the first sequence region (8).

the method comprising:
contacting a sample with a first primer (12) and a second primer (14), of which at least one hybridizes with the second region (10), capable of generating a first amplicon (15) that comprises a sequence of the second region (10), and detecting said first amplicon (15), wherein detecting said amplicon (15) indicates the presence of MRSA or genetic material thereof in the sample.

In embodiments of the method according to the invention the first (12) and/or second primers (14), and/or the probe (13) for detecting the first amplicon (15) hybridize to a sequence according to SEQ ID NO 1, 2 or 3, or a sequence of at least 80% sequence identity thereto.

In embodiments of the method according to the invention the first (12) and/or second primers (14), and/or the probe (13) for detecting the first amplicon (15) hybridize to a sequence according to SEQ ID NO 1, 2 or 3, or a sequence of at least 90%, or 95%, sequence identity thereto.

Hence, in one embodiment of the method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and

- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:
contacting a sample with a first primer (12) and a second primer (14), of which at least one hybridizes with the second region (10), capable of generating a first amplicon (15) that comprises a sequence of the second region (10), and detecting said first amplicon (15), comprising detecting the presence of a signal from an oligonucleotide probe (4, 13) that hybridizes to the sequence of the amplicon (15, 6), wherein the **first** (12) and/or **second** primers (14), and/or the **probe** (13) for detecting the first amplicon (15) **hybridize** to a sequence according to SEQ ID NO 1, 2 or 3, or a sequence of at least 80% sequence identity thereto, and wherein detecting said amplicon (15) indicates the presence of MRSA or genetic material thereof in the sample.

*Embodiments related to the sequences of the first and second primers:*

In embodiments of the method according to the invention the first primer (12) hybridizes with the first region (8) according to SEQ ID NO 4, 5 or 6, or a sequence of at least 80% sequence identity thereto, and the second primer (14) hybridizes with the second region (10) according to SEQ ID NO 1, 2 or 3, or a sequence of at least 80% sequence identity thereto.

In embodiments of the method according to the invention the first primer (12) hybridizes with the first region (8) according to SEQ ID NO 4, 5 or 6, or a sequence of at least 90%, or 95%, sequence identity thereto, and the second primer (14) hybridizes with the second region (10) according to SEQ ID NO 1, 2 or 3, or a sequence of at least 90%, or 95%, sequence identity thereto.

Hence, in embodiments the invention relates to a method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and

- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:

contacting a sample with a first primer (12) and a second primer (14), of which at least one hybridizes with the second region (10), capable of generating a first amplicon (15) that comprises a sequence of the second region (10), wherein the first primer (12) hybridizes with the first region (8) according to SEQ ID NO 4, 5 or 6, or a sequence of at least 80% sequence identity thereto, and/or the second primer (14) hybridizes with the second region (10) according to SEQ ID NO 1, 2 or 3, or a sequence of at least 80% sequence identity thereto, and

detecting said first amplicon (15), and wherein detecting said amplicon (15) indicates the presence of MRSA or genetic material thereof in the sample.

In embodiments of the method according to the invention the first primer (12) comprises or consists of a sequence according to SEQ ID NO 7, 8, 9, or 45 or a sequence of at least 80% sequence identity thereto.
In embodiments of the method according to the invention the first primer (12) comprises or consists of a sequence according to SEQ ID NO 7, 8, 9, or 45 or a sequence of at least 90%, or 95%, sequence identity thereto.
In embodiments of the method according to the invention the second primer (14) comprises or consists of a sequence according to SEQ ID NO 10, 11 or 12, or a sequence of at least 80% sequence identity thereto.
In embodiments of the method according to the invention the second primer (14) comprises or consists of a sequence according to SEQ ID NO 10, 11 or 12, or a sequence of at least 90%, Or 95%, sequence identity thereto.
Accordingly, in embodiments of the method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and

- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:

contacting a sample with a first primer (12) and a second primer (14), of which at least one hybridizes with the second region (10), capable of generating a first amplicon (15) that comprises a sequence of the second region (10), wherein the first primer (12) comprises or consists of a sequence according to SEQ ID NO 7, 8, 9, or 45 or a sequence of at least 80% sequence identity thereto, and/or the second primer (14) comprises or consists of a sequence according to SEQ ID NO 10, 11 or 12, or a sequence of at least 80% sequence identity thereto, and

detecting said first amplicon (15), wherein detecting said amplicon (15) indicates the presence of MRSA or genetic material thereof in the sample.

*Embodiments related to the sequences of the probes:*

In an embodiment of the method according to the invention the probe (13) for detecting the first amplicon (15) from the first (12) and second primers (14) comprises or consists of a sequence according to SEQ ID NO 13, 14 or 15, or a sequence of at least 80% sequence identity thereto.
In an embodiment of the method according to the invention the probe (13) for detecting the first amplicon (15) from the first (12) and second primers (14) comprises or consists of a sequence according to SEQ ID NO 13, 14 or 15, or a sequence of at least 90%, or 95%, sequence identity thereto.

Hence, in one embodiment the method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1), wherein the SCCmec sequence (16) comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and
- a second sequence region (10) comprising a sequence of a methicillin-resistant *Staphylococcus aureus* (MRSA) and a mecA gene (17), adjacent to said first region (8), thereby forming a boundary between said first and second regions (11),

the method comprising:

contacting a sample with a first primer (12) and a second primer (14), of which at least one hybridizes with the second region (10), capable of generating a first amplicon (15) that comprises a sequence of the second region (10), and detecting said first amplicon (15), comprising detecting the presence of a signal from an oligonucleotide probe (4, 13) that hybridizes to the sequence of the amplicon (15, 6), wherein the probe (13) for detecting the first amplicon (15) from the first (12) and second primers (14) comprises or consists of a sequence according to SEQ ID NO 13, 14 or 15, or a sequence of at least 80% sequence identity thereto, and wherein detecting said amplicon (15) indicates the presence of MRSA or genetic material thereof in the sample.

In another embodiment of the method according to the invention the probe (13) for detecting the first amplicon (15) from the first (12) and second primers (14) hybridizes to a sequence according to SEQ ID NO 16, 17 or 18, or a sequence of at least 80% sequence identity thereto.

In another embodiment of the method according to the invention the probe (13) for detecting the first amplicon (15) from the first (12) and second primers (14) hybridizes to a sequence according to SEQ ID NO 16, 17 or 18, or a sequence of at least 90%, or 95%, sequence identity thereto.

In embodiments of the method according to the invention the probe for detecting the second amplicon (6) from the third (3) and fourth primers (5) comprises or consists of a sequence according to SEQ ID NO 37 or 38, or a sequence of at least 80%, 90%, or 95%, sequence identity thereto, and/or

the probe for detecting the second amplicon (6) from the third (3) and fourth primers (5) hybridizes to a sequence according to SEQ ID NO 39, 40, or 41, or a sequence of at least 80%, 90%, or 95%, sequence identity thereto.

*Aspects and embodiments relating to oligonucleotides, sets and/or kits thereof:*

In another aspect the present invention relates to at least one oligonucleotide, wherein the oligonucleotide may be a first, a second, a third and/or a fourth primer, and/or a probe, and/or a target SA sequence, as described herein. The oligonucleotides, such as primers, probes and/or SA target sequences, described herein and above are, in embodiments, considered subject matter of the invention.

In embodiments, the oligonucleotide according to the invention is a first primer (12) as described herein. In embodiments, the oligonucleotide according to the invention is a second primer (14) as described herein. In embodiments, the oligonucleotide according to the invention is a third primer (3) as described herein. In embodiments the oligonucleotide according to the invention is a fourth primer (5) as described herein.

In embodiments of the present invention, the oligonucleotides (primers) according to the invention are suitable or configured to amplify, in a nucleic acid amplification reaction, such as PCR, a nucleic acid sequence of interest (target), such as a nucleic acid sequence associated with MRSA, if present in a sample, thereby generating an amplicon, e.g., (6) or (15).

In embodiments, the oligonucleotide is a first primer (12) or a second primer (14), which is able to hybridize with the second region (10), wherein the second region comprises a sequence of a methicillin-resistant *Staphylococcus aureus* (MRSA) and a mecA gene (17), adjacent to the first region (8).

In embodiments, the oligonucleotide is a first primer (12), which is able to hybridize with the first region (8), wherein the first region (8) comprises a sequence of a methicillin-sensitive *Staphylococcus aureus* (MSSA) positioned adjacent to a chromosomal orfX sequence (1).

In embodiments, the oligonucleotide according to the invention comprises or consists of a sequence that hybridizes to a sequence according to SEQ ID NO 1, 2 or 3, or a sequence of at least 80%, 90% or 95% sequence identity thereto. In embodiments said oligonucleotide is a first primer (12), a second primer (14) or a probe (13).

In embodiments, the oligonucleotide according to the invention comprises or consists of a sequence that hybridizes to a sequence according to SEQ ID NO 4, 5 or 6, or a sequence of at least 80%, 90% or 95% sequence identity thereto. In embodiments, the oligonucleotide according to the invention is a first primer (12), which comprises or consists of a sequence that hybridizes to a first region (8) according to SEQ ID NO 4, 5 or 6, or a sequence of at least 80%, 90% or 95% sequence identity thereto.

In embodiments, the oligonucleotide according to the invention comprises or consists of a sequence according to SEQ ID NO 7, 8, 9 or 45, or a sequence of at least 80%, 90% or 95% sequence identity thereto. In embodiments, the oligonucleotide according to the invention is a first primer (12) comprising or consisting of a sequence according to SEQ ID NO 7, 8, 9 or 45, or a sequence of at least 80%, 90% or 95% sequence identity thereto.

In embodiments, the oligonucleotide according to the invention comprises or consists of a sequence that hybridizes to a sequence according to SEQ ID NO 1, 2 or 3, or a sequence of at least 80%, 90% or 95% sequence identity thereto. In embodiments, the oligonucleotide according to the invention is a second primer (14), which comprises or consists of a sequence that hybridizes to the second region (10) according to SEQ ID NO 1, 2 or 3, or a sequence of at least 80%, 90% or 95% sequence identity thereto.

In embodiments, the oligonucleotide according to the invention comprises or consists of a sequence according to SEQ ID NO 10, 11 or 12, or a sequence of at least 80%, 90% or 95% sequence identity thereto. In embodiments the oligonucleotide according to the invention is a second primer (14), which comprises or consists of a sequence according to SEQ ID NO 10, 11 or 12, or a sequence of at least 80%, 90% or 95% sequence identity thereto.

In embodiments, the oligonucleotide according to the invention comprises or consists of a sequence that hybridizes to a sequence according to SEQ ID NO 39, 40, or 41, or a sequence of at least 80%, 90% or 95% sequence identity thereto. In embodiments, the oligonucleotide according to the invention is a third primer (3), which hybridizes to an orfX sequence (1) according to SEQ ID NO SEQ ID NO 39, 40, or 41, or a sequence of at least 80%, 90% or 95% sequence identity thereto.

In embodiments, the oligonucleotide according to the invention comprises or consists of a sequence according to SEQ ID NO 35 or 36, or a sequence of at least 80%, 90% or 95% sequence identity thereto. In embodiments, the oligonucleotide according to the invention is a third primer (3), which comprises or consists of a sequence according to SEQ ID NO 35 or 36, or a sequence of at least 80%, 90% or 95% sequence identity thereto.

In embodiments, the oligonucleotide according to the invention comprises or consists of a sequence that hybridizes to a sequence according to SEQ ID NO 42, 43 or 44, or a sequence of at least 80%, 90% or 95% sequence identity thereto. In embodiments, the oligonucleotide according to the invention is a fourth primer (5), which hybridizes with an MRSA SCCmec sequence (2), according to SEQ ID NO 42, 43 or 44, or a sequence of at least 80%, 90% or 95% sequence identity thereto.

In embodiments, the oligonucleotide according to the invention comprises or consists of a sequence according to one of SEQ ID NO 19-34, or a sequence of at least 80%, 90% or 95% sequence identity thereto. In embodiments, the oligonucleotide according to the invention is a fourth primer (5), which comprises or consists of a sequence according to one of SEQ ID NO 19-34, or a sequence of at least 80%, 90% or 95% sequence identity thereto.

In embodiments, the oligonucleotide according to the invention is a probe (4) or (13).

In embodiments, said probes ((4) or (13)) are able to hybridize to and/or detect an amplicon (generated) according to the invention ((6) or (15)). In preferred embodiments, the probe according to the invention enables through the hybridization to an amplicon ((6) or (15)) (generated) according to the invention the detection of a MRSA in a sample. In some embodiments the hybridization of the probe occurs during a nucleic acid amplification reaction, such as a qPCR or real-time PCR. The advantageous effect of the probes according to the invention is the detection of novel MRSA strain variants, especially if said probes are used together with at least one primer according to the invention.

There are no detection methods, probes and/or primers available in the prior art, that target the newly discovered boundary between a first and a second sequence region (the boundary between a MSSA- and MRSA-specific sequence). Accordingly, prior art methods and/or primers are unable to detect the newly discovered MRSA strains in standard PCR-based assays. On the contrary, the oligonucleotides according to the invention, such as, for example, the present probes surprisingly enable, especially in the context of the present method and/or in combination with at least one primer according to the invention, the detection of new MRSA-strains comprising a first and a second region, as described herein.

In embodiments, the oligonucleotide according to the invention comprises or consists of a sequence according to SEQ ID NO 13, 14 or 15, or a sequence of at least 80%, 90% or 95% sequence identity thereto. In preferred embodiments said oligonucleotide is a probe. In embodiments, the oligonucleotide according to the invention is a probe (13) for detecting a first amplicon (15) from the first (12) and second primers (14), wherein the probe comprises or consists of a sequence according to SEQ ID NO 13, 14 or 15, or a sequence of at least 80%, 90% or 95% sequence identity thereto.

In embodiments, the oligonucleotide according to the invention comprises or consists of a sequence that hybridizes to a sequence according to SEQ ID NO 16, 17, or 18, or a sequence of at least 80%, 90% or 95% sequence identity thereto. In preferred embodiments, said oligonucleotide is a probe. In embodiments, the oligonucleotide according to the invention is a probe (13) for detecting the first amplicon (15) from the first (12) and second primers (14), wherein the probe hybridizes to a sequence according to SEQ ID NO 16, 17, or 18, or a sequence of at least 80%, 90% or 95% sequence identity thereto.

In embodiments, the oligonucleotide according to the invention comprises or consists of a sequence that hybridizes to a sequence according to according to SEQ ID NO 39, 40, or 41, or a sequence of at least 80%, 90% or 95% sequence identity thereto. In preferred embodiments, said oligonucleotide is a probe. In embodiments, the oligonucleotide according to the invention is a probe (13) for detecting the second amplicon (6) from the third (3) and fourth primers (5), wherein the probe hybridizes to a sequence according to SEQ ID NO 39, 40, or 41, or a sequence of at least 80%, 90% or 95% sequence identity thereto.

In embodiments, the oligonucleotide according to the invention comprises or consists of a sequence according to SEQ ID NO 37 or 38, or a sequence of at least 80%, 90% or 95% sequence identity thereto. In preferred embodiments said oligonucleotide is a probe. In embodiments, the oligonucleotide according to the invention is a probe (13) for detecting the second amplicon (6) from the third (3) and fourth primers (5), wherein the probe comprises or consists of a sequence according to SEQ ID NO 37 or 38, or a sequence of at least 80%, 90% or 95% sequence identity thereto.

In embodiments of the invention a nucleotide sequence with at least 80% sequence identity to a specific nucleic acid sequence (a "target"), has preferably more than 80%, or more than 90% sequence identity, or, for example, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99,5 or 100% sequence identity to said specific nucleic acid sequence ("target"). Any mention of at least 80% or of at least 90% or of at least 95% may be replaced by any of the percentage values presented here.

In embodiments, the oligonucleotides according to the invention are preferably between 15 and 35 consecutive nucleotides (nt) or base pairs (bp) in length. Accordingly, a primer and/or probe according to the invention may have a length of preferably between 15 and 35 consecutive nt or bp. In embodiments, the oligonucleotides according to the invention may have a length between 20 and 30 bp or nt, or between 19 and 30 bp or nt. An oligonucleotide according to the invention may have a length of 5, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 bp or nt. A primer or probe according to the invention is preferably a DNA oligonucleotide.

If the oligonucleotide according to the invention is a probe, said probe may in embodiments comprise means that enable the detection of an amplicon the probes bind to. Said means may in embodiments comprise at least one fluorophore and/or at least one quencher. The probe according to the invention may in embodiments resemble or be similar to a hydrolysis- or TaqMan probe. In embodiments a fluorescence signal may be generated when the probe binds to a target nucleic acid sequence, such that the presence of a target nucleic acid sequence, e.g. an amplicon, can be detected.

In the context of the invention an oligonucleotide, such as the first (12) and/or second primers (14), and/or the probe (13), may hybridize to nucleic acid sequences (targets) exhibiting differences in nucleic acid sequence, so that for example one or more mismatches may occur between a primer and/or probe and the target/template nucleic acid sequence, e.g. of the SA to be detected. The primer(s) and/or probe(s) may in embodiments exhibit a similar $T_m$ and/or binding specificities for various SA strains, despite certain sequence variations, so that the function of the primer(s) and/or probe(s) in the context of the present invention is maintained, despite one or more mismatches.

In the context of the present invention an oligonucleotide, such as the primer(s) and/or probe(s), has a preferred $T_m$ of 45°-75°C, such as about 50°, 55°, 60°, 65° or 70°C. Suitable Tms may be elected by a skilled person. In preferred embodiments an oligonucleotide, such as the primer(s) and/or probe(s), binds specifically and reliably regardless of sequence variation of and/or mismatches to target SA (genome) regions.

For multiple copies of DNA molecules, such as oligonucleotides, the melting temperature ($T_m$) is generally defined as the temperature at which half of the DNA strands are in the double-helical state and half are in the random coil states. The melting temperature depends on both the length of the nucleic acid molecule (oligonucleotide), and the specific nucleotide sequence composition of that molecule. The melting temperature of an oligonucleotide primer can be calculated using various methods.

Theoretical and/or empirical measurements can be made for determining the $T_m$ of primers. Examples for theoretical measurement obtained from the Promega website relate to:

$$T_m = 4°C \times (\text{number of G's and C's in the primer}) + 2°C \times (\text{number of A's and T's in the primer})$$

This formula is valid for oligos <14 bases and assumes that the reaction is carried out in the presence of 50mM monovalent cations. For longer oligos, the formula below is used:

$$T_m = 64.9°C + 41°C \times (\text{number of G's and C's in the primer} - 16.4)/N \text{ (where N is the length of the primer)}$$

For example, Promega's T7 Promoter Primer (TAATACGACTCACTATAGGG) is a 20mer composed of 5 T's, 7 A's, 4 C's, and 4 G's. Thus, its melting temperature is calculated:

$$64.9°C + 41°C \times (8 - 16.4)/20 = 47.7°C$$

Examples of empirical determination of $T_m$ of a primer relate to determining the actual melting point, for example using a thermostatted cell in a UV spectrophotometer. If temperature is plotted vs. absorbance, an S-shaped curve with two plateaus will be observed. The absorbance reading halfway between the plateaus corresponds to $T_m$ (obtained from Sigma Aldrich). Other useful references for Tm calculation are Wallace, R.B.; Shaffer, J.; Murphy, R.F.; Bonner, J.; Hirose, T.; Itakura, K. Nucleic Acids Res. 6, 3543 (1979) and Howley, P.M; Israel, M.F.; law, M-F.; Martin,M.A. J. Biol. Chem. 254, 4876.

A preferred method for calculating Tm of the primers is according to the software Primer Premier 5. According to this method melting temperatures are based on nearest neighbour thermodynamic theory. The formulas are from the paper by Freier et al (1986 "Improved free-energy parameters for predictions of RNA duplex stability" Proc Natl Acad Sci USA 83 9373-9377). These are highly accurate nearest neighbour-based $T_m$ calculations. Several corrections have been incorporated into the formula to yield the most accurate results possible.

$$T_m = DH/(DS + R * \ln (C/4)) + 16.6 \log ([K+]/(1 + 0.7 [K+])) - 273.15$$

DH is the enthalpy for helix formation. DS is the entropy for helix formation. C is the DNA concentration. [K+] is the salt concentration. DH and DS for the respective oligo are calculated using the values of nearest-neighbour thermodynamics from Breslauer, K.J. (1986 "Predicting DNA duplex stability from the base sequence" Proc Natl Acad Sci USA 83 3746-3750). Since the thermodynamic values calculated by the method of Breslauer are at 1 M Na+, it was necessary to add a term to correct for salt concentration, [K+]. [K+] is calculated using both the monovalent ion concentration and the free Mg2+ concentration. The value of [K+] is determined using the formula:

$$[K+] = \text{Monovalent ion concentration} + 4 \times \text{Sq. root (Free Mg2+ ion concentration)} \times 1000$$

The salt concentration i.e., [K+] is represented as Total Na+ Equivalent and is updated according to the changes made in either Monovalent ion concentration or Free Mg2+ ion concentration. The default values of Monovalent ion concentration and Free Mg2+ ion concentration are 50.0 mM and 1.5 mM res., which gives a total salt concentration equal to 204.9 mM. In the formulation of Freier et al., c is the total molar concentration of the annealing oligos, when oligos are not self-complementary. It is neither the primer concentration nor the initial template concentration. It is based on the PCR product concentration. Since the concentration of template changes dramatically during the course of the PCR, it is difficult to define the value of "c". It has been empirically determined that setting the value of c = 250 pM, gives good results in a wide variety of PCR and sequencing reactions.

The annealing temperature ($T_a$) chosen for the amplification depends on the length and composition of the primer(s) and/or probe(s). Generally, an annealing temperature about 2 to 7 °C, for example 5°C, below the lowest $T_m$ of a pair of primers and/or of a probe is used. Such a strategy may be employed for the present method. Slightly higher $T_a$ may also be applied in order to increase the stringency of the reaction, for example $T_a$ of approximately the same $T_m$ as the primers may be applied. Therefore, the $T_a$ in the amplification reaction as described herein may be, in one embodiment, from 45 to 65°C, preferably from 55 to 65 °C, more preferably approximately 60 °C. Other $T_a$ values may be used depending on the primers applied.

In the meaning of the invention these ranges also include the values 55.0, 55.1, 55.2, 55.3, 55.4, 55.5, 55.6, 55.7, 55.8, 55.9, 56.0, 56.1, 56.2, 56.3, 56.4, 56.5, 56.6, 56.7, 56.8, 56.9, 57.0, 57.1, 57.2, 57.3, 57.4, 57.5, 57.6, 57.7, 57.8, 57.9, 58.0, 58.1, 58.2, 58.3, 58.4, 58.5, 58.6, 58.7, 58.8, 58.9, 59.0, 59.1, 59.2, 59.3, 59.4, 59.5, 59.6, 59.7, 59.8, 59.9, 60.0, 60.1, 60.2, 60.3, 60.4, 60.5, 60.6, 60.7, 60.8, 60.9, 61.0, 61.1, 61.2, 61.3, 61.4, 61.5, 61.6, 61.7, 61.8, 61.9, 62.0, 62.1, 62.2, 62.3, 62.4, 62.5, 62.6, 62.7, 62.8, 62.9, 63.0, 63.1, 63.2, 63.3, 63.4, 63.5, 63.6, 63.7, 63.8, 63.9, 64.0, 64.1, 64.2, 64.3, 64.4, 64.5, 64.6, 64.7, 64.8, 64.9 and 65° C, and all other values falling within the range.

Embodiments of the present invention are intended to be applied or used in clinical diagnostic applications, in particular PCR/qPCR or sequencing kits and/or applications and assays for MRSA diagnosis, which provide a fast and reliable MRSA diagnosis without the need for extensive analysis methods, nucleic acid sequencing and further tests. Accordingly, also the use of the present method for the detection of MRSA in a sample is intended and envisaged.

One aspect of the invention further relates to a set of oligonucleotides, comprising two or more of the oligonucleotides described herein in physical proximity. The oligonucleotides may be present in combination, in a single composition, or in separate vials or compositions, in physical proximity, for example in a kit, a combined packaging, or being offered together for combined purchase.

In embodiments, the set of oligonucleotides comprises:

- A set of oligonucleotides, each with a sequence that hybridizes to a sequence according to SEQ ID NO 1, 2 or 3, or a sequence of at least 80%, 90% or 95% sequence identity thereto,
- A set of oligonucleotides, each with a sequence that hybridizes to a sequence according to SEQ ID NO 4, 5 or 6, or a sequence of at least 80%, 90% or 95% sequence identity thereto,
- A set of oligonucleotides, each with a sequence according to SEQ ID NO 7, 8, 9 or 45, or a sequence of at least 80%, 90% or 95% sequence identity thereto,
- A set of oligonucleotides, each with a sequence according to SEQ ID NO 10, 11 or 12, or a sequence of at least 80%, 90% or 95% sequence identity thereto,
- A set of oligonucleotides, each with a sequence that hybridizes to a sequence according to SEQ ID NO 39, 40, or 41, or a sequence of at least 80%, 90% or 95% sequence identity thereto,
- A set of oligonucleotides, each with a sequence according to SEQ ID NO 35 or 36, or a sequence of at least 80%, 90% or 95% sequence identity thereto,
- A set of oligonucleotides, each with a sequence that hybridizes to a sequence according to SEQ ID NO 42, 43 or 44, or a sequence of at least 80%, 90% or 95% sequence identity thereto,
- A set of oligonucleotides, each with a sequence according to one of SEQ ID NO 19-34, or a sequence of at least 80%, 90% or 95% sequence identity thereto,
- A set of oligonucleotides, each with a sequence according to SEQ ID NO 13, 14 or 15, or a sequence of at least 80%, 90% or 95% sequence identity thereto,
- A set of oligonucleotides, each with a of a sequence that hybridizes to a sequence according to SEQ ID NO 16, 17, or 18, or a sequence of at least 80%, 90% or 95% sequence identity thereto,
- A set of oligonucleotides, each with a sequence that hybridizes to a sequence according to according to SEQ ID NO 39, 40, or 41, or a sequence of at least 80%, 90% or 95% sequence identity thereto, and/or
- A set of oligonucleotides, each with a sequence according to SEQ ID NO 37 or 38, or a sequence of at least 80%, 90% or 95% sequence identity thereto.

The present invention further relates to the use of an oligonucleotide, or an oligonucleotide set, according to the invention for the detection of a MRSA in a sample. In preferred embodiments the detection is accomplished by or in the context of the method described herein.

The present invention also relates to the use of an oligonucleotide according to the invention for the characterization of a MRSA in a sample. In preferred embodiments the characterization is accomplished by or in the context of the method described herein.

In another aspect the invention relates to a kit for performing the method according to the invention. A kit according to the invention can comprise any means necessary for performing the method according to the invention. In some embodiments the kit according to the invention may comprise at least one primer and/or at least one probe according to the invention. In embodiments, the kit comprises one or more of th4e oligonucleotides and/or sets thereof described herein.

In embodiments the kit according to the invention comprises at least a first and a second primer according to the invention and/or at least a third and a fourth primer according to the invention and optionally at least one probe. The kit optionally further comprises means for performing a nucleic acid amplification and/or detection reaction, such as PCR, reverse transcriptase PCR (RT-PCR), qPCR and/or real-time PCR.

The kit according to the invention enables in embodiments the detection of a methicillin-resistant *Staphylococcus aureus* (MRSA) strain or genetic material thereof in a sample by a nucleic acid amplification reaction. In preferred embodiments the kit enables the detection of a MRSA or MRSA strain, wherein the MRSA strain comprises a staphylococcal cassette chromosome mec (SCCmec) sequence (16) adjacent to a chromosomal orfX sequence (1), wherein the SCCmec sequence (16) preferably comprises:

- a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and
- a second sequence region (10) comprising a sequence of a methicillin-resistant *Staphylococcus aureus* (MRSA) and a mecA gene (17), adjacent to said first region (8), thereby forming a boundary between said first and second regions (11).

In another embodiment the kit enables the detection of a MRSA or MRSA strain, wherein the MRSA strain comprises a staphylococcal cassette chromosome mec (SCCmec) sequence (16) adjacent to a chromosomal orfX sequence (1), wherein the SCCmec sequence (16) comprises a sequence region (2) comprising a sequence of a methicillin-resistant *Staphylococcus aureus* (MRSA), the sequence (2) positioned adjacent to a chromosomal orfX sequence (1), thereby forming a boundary (7) between said sequence region (2) and orfX sequence (1).

In embodiments, preferably said kit comprises at least a third primer (3) comprising or consisting of a sequence according to SEQ ID NO 35 or 36, or a sequence of at least 80% sequence identity thereto, and/or at least a fourth primer (5) comprising or consisting of a sequence according to one of SEQ ID NO 19-34, or a sequence of at least 80% sequence identity thereto, and/or at least one probe (4) for detecting the second amplicon (6) from the third (3) and fourth primers (5), wherein the probe comprises or consists of a sequence according to SEQ ID NO 37 or 38 or a sequence of at least 80% sequence identity thereto, and/or wherein the probe hybridizes to a sequence according to SEQ ID NO 39, 40 and/or 41 or a sequence of at least 80% sequence identity thereto, and optionally means for nucleic acid amplification and/or detection.

In embodiments the kit for detecting a methicillin-resistant *Staphylococcus aureus* (MRSA) strain or genetic material thereof comprises:

- a first primer (12) comprising or consisting of a sequence according to SEQ ID NO 7, 8, 9 or 45, or a sequence of at least 80% sequence identity thereto,

- a second primer (14) comprising or consisting of a sequence according to SEQ ID NO 10, 11 or 12, or a sequence of at least 80% sequence identity thereto,

- a third primer (3) comprising or consisting of a sequence according to SEQ ID NO 35 or 36, or a sequence of at least 80% sequence identity thereto,

- a fourth primer (5) comprising or consisting of a sequence according to one of SEQ ID NO 19-34, or a sequence of at least 80% sequence identity thereto,

- at least one probe (13) for detecting the first amplicon (15) from the first (12) and second primers (14), wherein the probe comprises or consists of a sequence according to SEQ ID NO 13, 14 or 15, or a sequence of at least 80% sequence identity thereto, and/or wherein the probe hybridizes to a sequence according to SEQ ID NO 16, 17, or 18, or a sequence of at least 80% sequence identity thereto, and/or

- at least one probe (4) for detecting the second amplicon (6) from the third (3) and fourth primers (5), wherein the probe comprises or consists of a sequence according to SEQ ID NO 37 or 38 or a sequence of at least 80% sequence identity thereto, and/or
wherein the probe hybridizes to a sequence according to SEQ ID NO 39, 40 and/or 41 or a sequence of at least 80% sequence identity thereto,

- and optionally means for nucleic acid amplification and/or detection.

The present invention further relates to the use of the kit according to the invention for the specific detection of a MRSA in a sample. In preferred embodiments the detection is accomplished by or in the context of the method described herein.

The present invention also relates to the use of the kit according to the invention for the characterization of a MRSA in a sample. In preferred embodiments the characterization is accomplished by or in the context of the method described herein.

Preferably a subject, patient or host may be an organism, a cell culture of patient cells or cell lines, an animal, or a cell culture of animal cells or cell lines. In embodiments a subject or patient can be selected from the group comprising vertebrae, animals, livestock, mammals, humans, preferably mammal or human.

The method of the present invention is intended to be carried out using samples obtained from subjects or patients to be tested for the presence of MRSA. Samples to be tested can be obtained via various measures, for example from body fluids of subjects, such as blood or saliva. Tissue samples, such as skin samples, or nasal secretions may also be obtained and subsequently tested for the presence of MRSA using the invention described herein. Bacterial cultures may also be used, if for example the MRSA has been isolated and cultured before testing.

Accordingly, a sample or patient sample herein may be selected from the group comprising a liquid sample, a solid sample, an environmental sample, a biopsy, a liquid biopsy, a tissue sample, a cell culture sample or a swap-derived sample. Basically, any kind of sample that is suspected to contain MRSA or genetic material thereof. The sample may further comprise or be a bodily fluid, whole blood or blood parts, plasma, serum, cells, tissue, saliva, sputum, mucus, phlegm, semen, vaginal fluids, liquor or cerebrospinal fluid, urine or pleural effusions.

Embodiments and features of the invention described with respect to the method, the oligonucleotides and kits are considered to be disclosed with respect to each and every other aspect of the disclosure, such that features characterizing the methods, may be employed to characterize the oligonucleotides, or kit and vice-versa. The various aspects of the invention are unified by, benefit from, are based on and/or are linked by the common and surprising finding of the detection of novel MRSA strains, preferably comprising a MSSA-associated sequence adjacent to the orfX sequence and in between the orfX sequence and a MRSA-conferring sequence.

## DETAILED DESCRIPTION OF THE INVENTION

All cited documents of the patent and non-patent literature are hereby incorporated by reference in their entirety.

The current state of the art methods for detecting MRSA by PCR is based on the principle established by Hiramatsu (U S6,156,507) of determining the sequence of the boundary between the orfX region of the *Staphylococcus aureus* genome and the SCCmec cassette. This involves the formation of a PCR product consisting of sequences from orfX and the SCCmec cassette containing the sequence of the boundary region. Some subsequently developed state of the art methods are based on the determination of this genome structure.

However, the inventors recently found that there are MRSA strains that have another sequence at the junction between orfX and the SCCmec cassette that is identical to MSSA (non-MRSA) strains. Hence, with regard to these newly discovered MRSA strains, the prior art methods, e.g. like the principle described by Hiramatsu, are not able to reliably differentiate between MRSA, CoNS and MSSA, as they analyze a region of said MRSA strains, which resembles the genome of MSSA strains, leading to false-negative results. In the context of the medical relevance and dangers of methicillin-resistant SA such false-negative results pose a severe risk to public health and hospital or immunocompromised patients.

The present method provides an urgently needed solution to this problem. In newly identified MRSA strains, there exists a boundary between the MSSA sequence and the methicillin resistance-conferring region. This boundary is comprised within the SCCmec cassette, but not directly adjacent to the orfX-SCCmec cassette.

Methicillin, also known as meticillin, is a narrow-spectrum beta-lactam ($\beta$-lactam) antibiotic of the penicillin class. Like other beta-lactam antibiotics, methicillin acts by inhibiting the synthesis of bacterial cell walls. It inhibits cross-linkage between the linear peptidoglycan polymer chains that make up a major component of the cell wall of gram-positive bacteria. It does this by binding to and competitively inhibiting the transpeptidase enzyme (also known as penicillin-binding proteins (PBPs)). These PBPs crosslink glycopeptides (D-alanyl-alanine), forming the peptidoglycan cell wall. Methicillin and other $\beta$-lactam antibiotics are structural analogs of D-alanyl-alanine, and the transpeptidase enzymes that bind to them are sometimes called penicillin-binding proteins (PBPs). Methicillin is a penicillinase-resistant B-lactam antibiotic, as it is not bound and hydrolysed by penicillinase. Penicillinase is a bacterial enzyme produced by bacteria resistant to other B-lactam antibiotics which hydrolyses the antibiotic, rendering it nonfunctional. *Staphylococcus aureus* (SA, S. aureus, Staph aureus, Staph a. or S. aureus) is a Gram-positive round-shaped (coccal) bacterium, a member of the Bacillota, and is a usual member of the microbiota of the body, frequently found in the upper respiratory tract and on the skin. *Staphylococcus aureus* strains unable to resist or "sensitive"/"susceptible" to beta-lactam ($\beta$-lactam) antibiotics are classified as methicillin-susceptible or methicillin-sensitive *Staphylococcus aureus* (MSSA). Infections with MSSA can usually be successfully treated using beta-lactam antibiotics, as these inhibit bacterial cell wall biosynthesis through irreversible binding to the transpeptidase-domain of penicillin-binding proteins (PBPs). Historically, the acquisition of a SCCmec cassette comprising a mecA sequence in methicillin-sensitive S. aureus (MSSA) has given rise to a number of genetically different MRSA lineages.

Methicillin-resistant *Staphylococcus aureus* (MRSA) strains are usually resistant or insensitive to the antibiotic methicillin and most other antibiotics (multi-resistant). MRSA bacteria usually are found, e.g., in the nasal atrium, throat, armpits and groin. If MRSA bacteria, however, enter the body through wounds or mucous membranes they can cause severe infections. MRSA predominantly occur where antibiotics are used at a high rate, such as in hospitals.

Herein a "SCCmec sequence" refers to the "SCCmec", the "staphylococcal cassette chromosome mec", mobile genetic element of *Staphylococcus,* which usually carries the "mecA" (methicillin-resistant gene) and other functional genes by comprising the two components of the mec gene complex and the ccr gene complex. The mec-gene complex consists of mecA, the regulatory genes, and the associated insertion sequences and was classified into six different classes (A, B, C1, C2, D, and E), along with the ccr genes (ccrC or the pair ccrA and ccrB), which encode recombinases that mediate the integration and excision of SCCmec into and from the chromosome. Resistance of *staphylococci* to methicillin and all $\beta$-lactam antibiotics is associated with the presence of the mecA gene, which encodes the low affinity of a penicillin-binding protein, PBP2a, that is absent in susceptible or sensitive staphylococci. Hence, $\beta$-lactam antibiotics can no longer bind to the modified penicillin binding protein, which can continue to promote cell wall synthesis and hence proliferation of the MRSA bacteria.

Herein, a "chromosomal orfX sequence" refers to the nucleic acid sequence of the *staphylococcus* "orfX" gene, which is conserved among all *staphylococci.* Upon insertion of the staphylococcal chromosome cassette mecA (SCCmec) its entire sequence is usually preserved. The function of orfX has not been determined, yet.

The present invention relates in one aspect to PCR-based methods when referring to nucleic acid amplification and/or to the detection reaction. PCR is one version of a nucleic acid amplification reaction und is as such known in the art and modifications of, or particular variations on, known PCR reactions or methods are included in the scope of the invention when used to carry out the method described herein. PCR relies on thermal cycling, consisting of cycles of repeated heating and cooling of the reaction for DNA melting (separation of the double strand) and enzymatic replication of the DNA. Primers relate to short DNA oligonucleotides that contain sequences complementary to the target region. A DNA polymerase is applied that facilitates selective and repeated amplification. As PCR progresses, the DNA generated is used as a template for replication, enabling a chain reaction in which the DNA template is exponentially amplified. As a result of a PCR reaction, at least one "amplicon" is generated. An amplicon is a piece of DNA or RNA that is the source and/or product of amplification or replication events. In the context of nucleic acid amplification, amplification refers to the generation of one or more copies of a genetic sequence or target sequence, e.g. the amplicon. As it refers to the product of an amplification reaction, amplicon is used interchangeably with common laboratory terms, e.g. "PCR product".

In the case of RNA-analysis said RNA can first be reverse-transcribed into DNA (cDNA) using a reverse transcriptase enzyme (RT-polymerase). A reverse transcription of RNA to cDNA can be performed before or in the context of a PCR reaction, such as in the context of a RT-PCR reaction. A certain RNA sequence of interest may be, after reverse-transcription into cDNA, subsequently detected as described herein, for example in the context of a (real-time / qPCR) PCR reaction.

One preferred embodiment relates to real-time PCR or quantitative real-time PCR (qPCR), as it allows not only the detection, but the quantification of the amplified target in real-time. Hence, in embodiments of the present invention the amplification and/or detection reaction may be or comprise a real-time or qPCR reaction. The term "real-time PCR" is intended to mean any amplification technique which makes it possible to monitor the progress of an ongoing amplification reaction as it occurs (i.e. in *real time*). Data is therefore collected during the exponential phase of the PCR reaction, rather than at the end point as in conventional PCR. Measuring the kinetics of the reaction the early phase of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods may also be applied, and use separation methods, such as agarose gels, for detection of PCR amplification at the final phase of or endpoint of the PCR reaction. For qPCR no post-PCR processing of the unknown DNA sample is necessary as the quantification occurs in real-time during the reaction. Furthermore, an increase in reporter fluorescent signal is directly proportional to the number of amplicons generated.

In another preferred embodiment the invention relates to a method, wherein the amplification and/or detection reaction is a multiplex PCR with more than one pair of primers and/or detection probes. The multiplex PCR is a variant of the standard PCR in which two or more loci are simultaneously amplified and/or detected in the same reaction, by including more than one pair of primers and/or probes in the reaction.

Herein the term "oligonucleotide" may refer to or comprise a primer (sequence) or a probe (sequence) of a certain length of consecutive nucleotides. In the context of the present invention the terms "oligonucleotide" and "primer", or "oligonucleotide" and "probe" may be used interchangeably. Oligonucleotides are short DNA or RNA molecules (oligomers). Oligonucleotides are generally characterized by the sequence of (consecutive) nucleotide residues that make up the molecule. The length of the oligonucleotide is usually denoted by "-mer". For example, an oligonucleotide of six nucleotides (nt) is a hexamer, while one of 25 nt would usually be called a "25-mer". Oligonucleotides usually readily bind, in a sequence-specific manner, to their respective complementary oligonucleotides, DNA, or RNA to e.g. form duplexes. Examples of procedures that use oligonucleotides include DNA microarrays, Southern blots, ASO analysis, fluorescent in situ hybridization (FISH) or any type of PCR amplification and/or detection.

The term "primer" as used herein refers to an "oligonucleotide", whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an inducing agent such as DNA polymerase and at a suitable temperature and pH. The primer or oligonucleotide is preferably single stranded for maximum efficiency in amplification but may alternatively be double stranded. If double stranded, the primer is first treated to separate its strands before being used to prepare extension products. Preferably, the primer is an oligodeoxyribonucleotide. The primer or oligonucleotide must be sufficiently long to prime the synthesis of extension products in the presence of the inducing agent. The exact lengths of the primers will depend on many factors, including temperature, source of primer and use of the method. For example, for diagnostics applications, depending on the complexity of the target sequence, the oligonucleotide primer typically contains 15-25 or more nucleotides, although it may contain fewer nucleotides.

The oligonucleotides, probes or primers herein are selected to be "substantially" complementary to the different strands of each specific sequence to be amplified. This means that the oligonucleotides, probes or primers must be sufficiently complementary to hybridize with their respective strands. Therefore, the sequences do not need to reflect the exact complementary sequence of the template. For example, a non-complementary nucleotide fragment (oligonucleotide) may be attached to the 5' end of a primer, with the remainder of a primer sequence being complementary to the strand. Alternatively, non-complementary bases or longer sequences can be interspersed into a primer, provided that a primer sequence has sufficient complementarity with the sequence of the strand to be amplified to hybridize therewith and thereby form a template for synthesis of the extension product of the other primer.

Hybridization is the process of establishing a non-covalent, sequence-specific interaction between two or more complementary strands of nucleic acids into a single hybrid, which in the case of two strands is referred to as a duplex or DNA double strand in the case of DNA. Oligonucleotides, primer, probes, DNA, or RNA will bind to their complement under normal conditions, so two perfectly complementary strands will bind to each other readily. Due to the different molecular geometries of the nucleotides, a single inconsistency between the two strands will make binding between them less energetically favorable. The hybrids may be dissociated by thermal denaturation, also referred to as melting. Here, the solution of hybrids is heated to break the hydrogen bonds between nucleic bases, after which the two strands separate. In the absence of external negative factors, the processes of hybridization and melting may be repeated in succession indefinitely, which lays the ground for polymerase chain reaction.

Alternative nucleic acid amplification technologies may also be applied that are known in the field of molecular biology. The polymerase chain reaction (PCR) is the most widely used method for DNA amplification, however, PCR requires a thermocycling machine to separate two DNA strands and then amplify the required fragment. Novel developments in molecular biology demonstrate the possibility of amplifying DNA in isothermal conditions without the need of a thermocycling apparatus. Isothermal approaches for amplification are therefore also included in the scope of the present invention. There are several types of isothermal nucleic acid amplification methods such as transcription mediated amplification, nucleic acid sequence-based amplification, signal mediated amplification of RNA technology, strand displacement amplification, rolling circle amplification, loop-mediated isothermal amplification of DNA, isothermal multiple displacement amplification, helicase-dependent amplification, single primer isothermal amplification, and circular helicase-dependent amplification (see Gill et al, Nucleosides Nucleotides Nucleic Acids. 2008 Mar;27(3):224-43, for a review).

Loop mediated isothermal amplification (LAMP) is one example of isothermal amplification that relates to a single tube technique for the amplification of DNA. LAMP enables nucleic acid amplification which uses a single temperature incubation thereby obviating the need for expensive thermal cyclers. Detection of amplification product can be by photometry for turbidity caused by increasing quantity of Magnesium pyrophosphate in solution or with addition of SYBRgreen, a color change can be seen without equipment. Also, in-tube detection of DNA amplification is possible using manganese loaded calcein which starts fluorescing upon complexation of manganes by pyrophosphate during in vitro DNA synthesis. LAMP is a relatively new DNA amplification technique, which due to its simplicity, ruggedness, and low cost could provide major advantages. In LAMP, the target sequence is amplified at a constant temperature of 60 - 65 °C using either two or three sets of primers and a polymerase with high strand displacement activity in addition to a replication activity. LAMP can also be quantitative.

The term "DNA polymerase" describes an enzyme frequently used in the polymerase chain reaction. Such enzymes include the Taq polymerase, a thermostable DNA polymerase named after the thermophilic bacterium Thermus aquaticus. Other thermostable polymerases are e.g. the Pfu DNA polymerase, which has been isolated from the hyperthermophilic bacterium *Pyrococcus furiosus* and has a 3'-5' exonuclease proofreading activity. Two DNA polymerases are mentioned exemplarily but do not represent limitations to the invention.

In the context of the present invention the detection of a certain nucleic acid sequence (target), such as a specific amplicon and/or MRSA-specific nucleic acid sequence, is in preferred embodiments achieved by using hybridization probes specific for a nucleic acid sequence, or an amplicon. Such a hybridization probe, or short "probe" might be used in embodiments in a nucleic acid amplification reaction. In the context of the present invention, the term "probe" or "hydrolysis probe" refers to a hybridization probe, which is a molecule for the detection of a nucleic acid sequence, such as an amplicon. It can be used in samples containing nucleic acids to detect the presence of a nucleotide or nucleic acid sequence that is at least partially complementary to the sequence in the probe, for example by real-time PCR or q-PCR/real-time PCR. The probe thereby hybridizes to single-stranded nucleic acid whose base sequence allows probe-target base pairing due to complementarity between the probe and the target. For detection the probe might be labeled (or "tagged") with one or more molecular markers of either radioactive and/or preferably fluorescent and/or a quencher molecule. Commonly used radioactive markers are 32P (a radioactive isotope of phosphorus incorporated into the phosphodiester bond in the probe DNA) or Digoxigenin, which is a non-radioactive, antibody-based marker. Detection of sequences with moderate or high similarity depends on how stringent the hybridization conditions are applied. High stringency, such as high hybridization temperature and low salt in hybridization buffers, permits only hybridization between nucleic acid sequences that are highly similar, whereas low stringency, such as lower temperature and high salt, allows hybridization when the sequences are less similar. During real-time or qPCR, hydrolysis probes comprising a fluorescent marker and a corresponding quencher can be used for detection of a nucleic acid sequence. During amplification of a nucleic acid sequence to which a hydrolysis probe is binding by means of primers that lie outside the target sequence, the amplifying polymerase can degrade or hydrolyze the probe from the 5'-end. This leads to the separation of the fluorescence marker and the corresponding quencher that were initially localized in close proximity to each other attached to the probe. Through separation of the fluorescent marker and the quencher, a detectable change in a signal can be generated. Hydrolysis probes are also called 5'-exonuclease probes or TaqMan probes and are well established molecular biology tools that are well known to the person skilled in the art. Further techniques such as molecular beacon probe technology, Scorpion probe technology, nanoparticle probe technology or Amplifluor probe technology may also be used herein for the detection of an amplicon or nucleic acid sequence of interest, such as an MRSA-specific sequence.

In the context of the present invention, an oligonucleotides or probe might be labelled with a fluorophore and optionally a quencher that interacts with said fluorophore. A "fluorophore" (or fluorochrome, similarly to a chromophore) is a fluorescent compound that can re-emit light upon light excitation. Fluorophores for use in the context of the oligonucleotides of the present invention can be selected from, without being limited to, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3- 25 coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 30 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Redmond Red, BODIPY 581/591, Cy3.5, Cal Red/Texas Red, BODIPY TR-X, BODIPY 630/665-X, Pulsar-650, Quasar-670/Cy5.

Herein "quenching" refers to any process which decreases the fluorescence intensity of a given substance. Quenching is the basis for Förster resonance energy transfer (FRET) assays. FRET is a dynamic quenching mechanism wherein energy transfer occurs while the donor is in an excited state. A "quencher" is a molecule, which quenches the fluorescence emitted by the fluorophore when excited by the cycler's light source via FRET. Quenchers that might be used in the context of the present invention can be selected from the group comprising, but without being limited to, DDQ-I, 22 Dabcyl, Eclipse, Iowa Black FQ, BHQ-1, QSY-7, BHQ-2, DDQ-II, Iowa Black RQ, QSY-21, BHQ- 3, QSY-35, BHQ-0, QSY-9, ElleQuencher, Iowa Black. The one skilled in the art can routinely choose suitable reporter-quencher combinations.

The term "nucleic acids" refers to nucleic acid molecules including, without limitation, DNA, ssDNA, dsDNA, RNA, mRNA, tRNA, lncRNA, ncRNA, microRNA, siRNA, rRNA, sgRNA, piRNA, rmRNA, snRNA, snoRNA, scaRNA, gRNA or viral RNA.

As used herein, the terms "gene" or "coding sequence," is meant to refer broadly to a DNA region (the transcribed region) which encodes a protein. A coding sequence is transcribed (DNA) and translated (RNA) into a polypeptide when placed under the control of an appropriate regulatory region, such as a promoter. A gene may comprise several operably linked fragments, such as a promoter, a 5'-leader sequence, a coding sequence and a 3'-non-translated sequence, comprising a polyadenylation site. The phrase "expression of a gene" refers to the process wherein a gene is transcribed into an RNA and/or translated into an active protein.

The term "nucleic acid amplification reaction" refers to any method comprising an enzymatic reaction, which allows the amplification of nucleic acids and is in some embodiments of the invention a polymerase chain reaction (PCR). Another embodiment relates to real time PCR or quantitative PCR (q-PCR), as it allows the quantification of the amplified nucleic acid, e.g. an amplicon, in real-time.

The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed.

Traditional PCR methods may also be applied herein, and use separation methods, such as agarose gels, for detection of PCR amplification at the final phase of or endpoint of the PCR reaction. For qPCR no post-PCR processing of the unknown DNA sample is necessary as the quantification occurs in real-time during the reaction. Furthermore, an increase in reporter fluorescent signal is directly proportional to the number of qPCR amplicons generated. Real-time PCR technique can be classified by the chemistry used to detect the PCR product, specific or non-specific fluorochromes. A non-specific DNA-binding dye binds to all double stranded (ds) DNA in PCR, causing fluorescence of the dye. An increase in DNA product during PCR therefore leads to an increase in fluorescence intensity measured at each cycle. In the context of the present invention in preferred embodiments the specific detection of PCR production can be achieved by using fluorescent reporter probes, which detect only the DNA containing the sequence complementary to the probe. Therefore, use of the reporter probe significantly increases specificity, and enables performing the technique even in the presence of other dsDNA. Using different-colored labels, fluorescent probes can be used in multiplex assays for monitoring several target sequences in the same tube.

The specificity of fluorescent reporter probes, e.g. probes according to the invention as described herein, also prevents interference of measurements caused by primer dimers, which are undesirable potential by-products in PCR. The method relies on a DNA-based probe with a fluorescent reporter at one end and a quencher of fluorescence at the opposite end of the probe. The close proximity of the reporter to the quencher prevents detection of its fluorescence; breakdown of the probe through hydrolysis by the 5' to 3' exonuclease activity of the polymerase used for the amplification reaction breaks the reporter-quencher proximity and thus allows unquenched emission of fluorescence, which can be detected after excitation with a laser. An increase in the product targeted by the reporter probe at each PCR cycle therefore causes a proportional increase in fluorescence due to the breakdown of the probe and release of the reporter. Due to the fact that this kind of assay is based on the 5'-exonuclease activity of the polymerase it is usually also called "5'-exonuclease assay". The before described detection mechanism is preferably performed in the context of a qPCR or real-time PCR. In another embodiment the invention relates to a method, wherein the detection comprises a multiplex PCR with more than one pair of primers. The multiplex PCR is a variant of the standard PCR in which two or more loci are simultaneously amplified in the same reaction, by including more than one pair of primers in the reaction.

As used herein, the term "hybridization" is used in reference to the pairing of complementary nucleic acids. Hybridization and the strength of hybridization (e.g., the strength of the association between the nucleic acids) is impacted by such factors as the degree of complementary between the nucleic acids, stringency of the conditions involved, the $T_m$ (melting temperature) of the formed hybrid, and the G:C ratio within the nucleic acids. As used herein the term "stringency" is used in reference to the conditions of temperature, ionic strength, and the presence of other compounds such as organic solvents, under which nucleic acid hybridizations are conducted. In an illustrative example, hybridization under "highly stringent condition" can mean hybridization at 65 0C in 5X SSPE and 50% formamide and washing at 65 oC in 0.5X SSPE. In another illustrative example "highly stringent condition" can mean hybridization at 55°C in a hybridization buffer consisting of 50% formamide (vol/vol); 10% dextran sulfate; 1 × Denhard"s solution; 20 mM sodium phosphate, pH 6.5; 5 x SSC; and 200 μg of salmon sperm DNA per ml of hybridization buffer for 18 to 24 hours, and washing four times (5 min each time) with 2 x SSC; 1% SDS at room temperature and then washing for 15 min at 50-55°C with 0.1 × SSC. In another illustrative example Conditions for high stringency hybridization are described in Sambrook et al., "Molecular Cloning: A Laboratory Manual", 3rd Edition, Cold Spring Harbor Laboratory Press, (2001), incorporated herein by reference. In some illustrative aspects, hybridization occurs along the full-length of the nucleic acid. Detection of highly stringent hybridization in the context of the present invention indicates strong structural similarity or structural homology (e.g., nucleotide structure, base composition, arrangement or order) to, e.g., the nucleic acids provided herein.

The term "target sequence" refers to the nucleic acid sequence that is to be detected by the probe or a set of primers (e.g. a sequence associated or indicative of a MRSA). The nucleic acid target sequence comprises a nucleic acid sequence, which is at least partially complementary to the nucleic acid sequence comprised in the probe. A probe according to the present invention comprises an oligonucleotide sequence complementary to the region of the nucleic target acid sequence. Said oligonucleotide sequence of the probe comprises, consists or essentially consists of 5 to 100 bases, preferably 5 to 50, 10 to 40, 12 to 38, 13 to 35, 14 to 32, 15 to 28, 16 to 26, 17 to 25, 18 to 24, 19 to 23, 20 to 22, 21 to 22, or most preferably 22 bases. The length of the probe is therefore preferably between 10 and 40 nucleotides in length, more preferably 10, 11 12, 13, 14, 15, 16, 25 17, 18, 19, 20, 21, 22, 23, 24, 25, 26, 27, 28, 29, 30, 31, 32, 34, 3,5 36, 37, 38, 39, or 40 nt.

In embodiments, a nucleic acid molecule, such as an oligonucleotide, primer or probe may have, a 0 to 10 nucleotides addition or deletion at the 5' or 3' terminus of a sequence, with reference to the specific sequences provided herein. As used herein the term "a 0 to 10 nucleotides addition or deletion at the 5' or 3' terminus of a sequence" means that the nucleic acid may have a) 0, 1, 2, 3, 4, 5, 6, 7, 8, 9 or 10 additional nucleotides at its 5' terminus and 0, 1, 2, 3, 4, 5, 6, 7, 8, 9 or 10 nucleotides deleted at its 3' terminus or b) 0, 1, 2, 3, 4, 5, 6, 7, 8, 9 or 10 additional nucleotides at its 3' terminus and 0, 1, 2, 3, 4, 5, 6, 7, 8, 9 or 10 nucleotides deleted at its 5' terminus, c) 0, 1, 2, 3, 4, 5, 6, 7, 8, 9 or 10 additional nucleotides at its 5' terminus and 0, 1, 2, 3, 4, 5, 6, 7, 8, 9 or 10 additional nucleotides at its 3' terminus or d) 0, 1, 2, 3, 4, 5, 6, 7, 8, 9 or 10 nucleotides deleted at its 5' terminus and 0, 1, 2, 3, 4, 5, 6, 7, 8, 9 or 10 nucleotides deleted at its 3' terminus.

As used herein, the term "complementary" refers to the ability of purine and pyrimidine nucleotide sequences to associate through hydrogen bonding to form double- stranded nucleic acid molecules. Guanine and cytosine, adenine and thymine, and adenine and uracil are complementary and can associate through hydrogen bonding resulting in the formation of doublestranded nucleic acid molecules when two nucleic acid molecules have "complementary" sequences. The complementary sequences can be DNA or RNA sequences. The complementary DNA or RNA sequences are referred to as a "complement". Complementary may be "partial" in which only some of the nucleic acid bases are matched according to the base pairing rules, or, there may be "completely" or "total" complementary between the nucleic acids.

As used herein, a "percent (%) sequence identity" with respect to a specific "reference" nucleic acid sequence (e.g. of nucleic acid sequences SEQ ID NO 1-45) is defined as the percentage of nucleotides in a certain sequence that are identical with the nucleotides in the "reference" nucleic acid sequence, after aligning the sequences and introducing gaps, if necessary, to achieve the maximum percent sequence identity, and not considering any conservative substitutions as part of the sequence identity. Alignment for purposes of determining percent nucleic acid sequence identity can be achieved in various ways that are within the skill in the art, for instance, using publicly available computer software programs. Software such as BLAST or Clustal enable such sequence alignments and calculation of percent identity. As used herein, the percent homology between two sequences is equivalent to the percent identity between the sequences. Determination of percent identity or homology between sequences can be done, for example, by using the GAP program (Genetics Computer Group, software; now available via Accelrys on http://www.accelrys.com), and alignments can be done using, for example, the ClustalW algorithm (VNTI software, InforMax Inc.). A sequence database can be searched using the nucleic acid sequence of interest. Algorithms for database searching are typically based on the BLAST software (Altschul et al., 1990). In some embodiments, the percent homology or identity can be determined along the full-length of the nucleic acid.

A "fluorophore" (or fluorochrome, similarly to a chromophore) is a fluorescent chemical compound that can re-emit light upon light excitation. Fluorophores for use as labels in constructing labeled probes of the invention comprise, without claiming to be exhaustive, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3-coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Redmond Red, BODIPY 581/591, Cy3.5, Cal Red/Texas Red, BODIPY TR-X, BODIPY 630/665-X, Pulsar-650, Quasar-670/Cy5. "Quenching" refers to any process which decreases the fluorescence intensity of a given substance. Quenching is the basis for Förster resonance energy transfer (FRET) assays. FRET is a dynamic quenching mechanism because energy transfer occurs while the donor is in the excited state. A "quencher" is a molecule, which quenches the fluorescence emitted by the fluorophore when excited by the cycler's light source via FRET.

Herein the terms "subject", "patient" or "host" may be used interchangeably. Herein as subject, patient or host may be an organism, a cell culture of patient cells or cell lines, an animal, or a cell culture of animal cells or cell lines. Herein a subject or patient refers to a species from which a sample is taken, and/or whose biological material makes up the majority of the biological material of a sample. A subject or patient can be selected from the group comprising vertebrae, animals, livestock, mammals, humans, preferably mammal or human.

The instant disclosure also includes kits, packages and multi-container units containing the herein described pharmaceutical compositions, active ingredients, and/or means for administering the same for use in the prevention and treatment of diseases and other conditions in mammalian subjects.

**FIGURES**

The invention is further described by the following figures. These are not intended to limit the scope of the invention but represent preferred embodiments of aspects of the invention provided for greater illustration of the invention described herein.

Brief description of the figures:

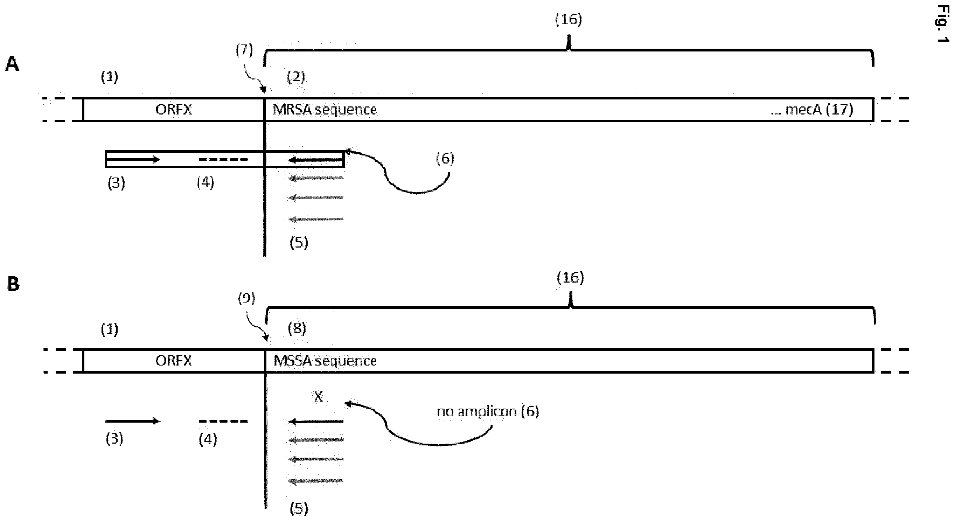

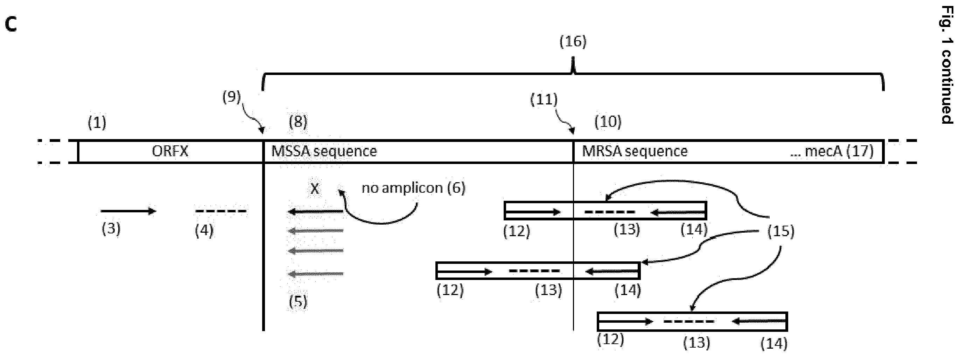

**Figure 1:** Scheme of the SCCmec-orfX genome regions of MRSA strains to be detected by embodiments of the present invention.

**Detailed description of the figures:**

**Figure 1:**

**Figure 1A** depicts embodiments of the present MRSA detection method using primers (5) that bind to and amplify the region spanning the boundary (7) between orfX (1) and the methicillin-resistant *Staphylococcus aureus* (MRSA) sequence region (2), (comprised within a SCCmec sequence (16)). This embodiment of the present method is able to detect MRSA strains where the MRSA sequence is located directly adjacent to the orfX cassette. The scheme depicts a genomic region of MRSA where the orfX gene region (1) is located adjacent to the SCCmec sequence (16) region comprising a MRSA sequence (2) and a mecA encoding sequence (17). The boundary (7) between the orfX region and the MRSA sequence can be detected and/or amplified using at least one third primer (3) and at least one fourth primer (5), wherein the third primer (3) binds preferably to the orfX sequence (or cassette) and the fourth primer (5) binds specifically to a MRSA-specific sequence (2), such that the generated amplicon (6) spans the boundary region (7). In embodiments the detection can be achieved using a probe (4) specific for the generated amplicon (6), which spans the boundary (7) between the orfX sequence and the MRSA sequence in the genome of the respective MRSA strain. In embodiments the probe (4) may be specific for the (conserved) genomic orfX region, such that a probe sequence can be used to detect amplicons of different MRSA strains and/or different MRSA sequence varieties.

**Figure 1B** depicts an embodiment of the invention, where a SCCmec sequence (16) region comprises a methicillin-sensitive *Staphylococcus aureus* (MSSA; (8)) sequence (and not a mecA sequence/gene), which is located adjacent to the orfX cassette (1) (and forms a boundary (9) with it). In said embodiments, no amplification (6) occurs with a MRSA-specific fourth primer (5) and a third primer (3), which binds to the orfX sequence. Accordingly, no amplicon would be detected using a specific probe (4).

**Figure 1C** depicts embodiments of the present MRSA detection method using primers (12,14) that bind to and amplify a region spanning the boundary (10) between a MSSA sequence (8) and a MRSA sequence region (10). In the depicted embodiment the SCCmec sequence (16) adjacent to the chromosomal orfX sequence (1) comprises a first sequence region (8) comprising a MSSA-specific sequence (8), which is located adjacent to the chromosomal orfX sequence (1) and forms a boundary (9) between said first sequence region (8; comprising a MSSA sequence) and the orfX sequence (1). The depicted SCCmec sequence also comprises a second sequence region (10) comprising a sequence of a MRSA and a mecA gene (17), that is adjacent to the first region (8) and forms a boundary with the first region (11). MRSA- specific fourth primer (5) cannot bind to the MSSA sequence adjacent to the orfX region. Accordingly, an amplification reaction with the third primer (3) and a fourth primer (5) would generate no amplicon (6) and would not be able to detect the presence of the MRSA-encoding sequence (10) in the represented MRSA strain. However, in the present embodiment a first primer (12) and a second primer (14), of which at least one hybridizes to the second region (10), are capable of generating a first amplicon (15) that comprises a sequence of the second region (10) and in preferred embodiments also the boundary (11) between the first and the second region. Said first MRSA-specific amplicon (15) could in embodiments be detected using a specific probe (13). In summary, the method of the present invention is able to detect the presence of genetically different MRSA strains or genetic material thereof in a sample, which could not be detected by prior art methods, which usually target the boundary (9) adjacent to the orfX region (1), which was described first by Hiramatsu.

*Table 1: Sequences referred to herein:*

| SEQ ID NO | Sequence | Comments |
|---|---|---|
| 1 | CCGCTTCATAAAGGGATTTTGAATGTATCAGAAC ATATGAGGTTTATGTGAATTGCTGTTATGTTTTTA AGAAGCTTATCATAAGTAATGAGGTTCATGATTTT TGACATAGTTAGCCTCCGCAGTCTTTCATTTCAA GTAAATAATAGCGAAATATTCTTTATACTGAATAC TTATAGTGAAGCAAAGTTCTAGCTTTGAGAAAATT CTTTCTGCAACTAAATATAGTAAATTACGGTAAAA TATAAATAAGTACATATTGAAGAAAATGAGACATA ATATATTTTATAATAGGAGGGAATTTCAAATGATA GACAACTTTATGCAGGTCCTTAAATTAATTAAAGA GAAACGTACCAATAATGTAGTTAAAAAATCTGATT GGGATAAAGGTGATCTATATAAAACTTTAGTCCAT GATAAGTTACCCAAGCAGTTAAAAGTGCATATAA AAGAAGATAAATATTCAGTTGTAGGGAAGGTTGC TACTGGGAACTATAGTAAAGTTCCTTGGATTTCAA TATATGATGAGAATATAACAAAAGAAACAAAGGA TGGATATTATTTGGTATATCTTTTTCATCCGGAAG GAGAAGGCATATACTTATCTTTGAATCAAGGATG GTCAAAGATAAGTGATATGTTCCGCGGGATAAA AATGCTGCAAAACAAAGAGCATTAACTTTATCTTC CGAACTCAATAAATATATTACATCAAATGAATTTA ATACTGGAAGATTTTATTACGCAGAAAATAAAGAT TCATCTTATGATTTAAAAAATGATTATCCATCAGG ATATTCTCATGGATCAATAAGATTCAAATATTATG ATTTGAATGAAGGATTCACAGAAGAAGATATGCT AGAGGATTTAAAGAAATTTTTAGAACTATTTAATG AATTAGCTTCAAAAGTTACAAAAACATCCTATGAT AGCTTGGTCAATAGCATAGACG | Second sequence region, wherein the 5' nt is adjacent to the 3' end of the first sequence region.<br>Sequence adjacent to and directly 3' (after) Boundary 8 (sample MFD5994) |
| 2 | TCATTTCGCCATTTTCCTACATTTTATAACCGCCA TTTACATGAATCAAAGGTAAAAGAAAAAGAAAAAA GAAAGCAACAAAGAGAAGAAGAAATAGAAAAAAG GAGAATAGCAAAAGAAGAGCAAGAAAAGCGTGA TGTTTGGATTAAAGAGAATGTATTTAAAATTATAG TAAATGAGGAAGGTAAACGAATAATTTTTTGTCCT AAATGTGGTAGTGAGAATAATGTAACTGCTACTA CTTGGAACGTAGATGAAGCTGAAAATAGTGAGGA TATTGGAACATATTTTAAGAGAACTTGTAATTTAT GCGGACATTCTCAAAAATTTAATAAAGATAACCCT AAAAAAATTTATGAATAGGAATAAAGAATTGATAC ATTTTCTGGATAAGTGAAAAAGACAAATTCTATTG AAATAATATAGAAATTGTCTTTAATGTTAGAATTTA TAAAACCTCATTATCAACCGATACGCAGAAGCAT ATCATAAATGATGCGGTTTTTTCAGCCGCTTCATA AAGGGATTTTGAATGTATCAGAACATATGAGGTT TATGTGAATTGCTGTTATGTTTTTAAGAAGCTTAT CATAAGTAATGAGGTTCATGATTTTTGACATAGTT AGCCTCCGCAGTCTTTCATTTCAAGTAAATAATAG CGAAATATTCTTTATACTGAATACTTATAGTGAAG CAAAGTTCTAGCTTTGAGAAAATTCTTTCTGCAAC | Second sequence region, wherein the 5' nt is adjacent to the 3' end of the first sequence region.<br>Sequence adjacent to and directly 3' (after) Boundary 23 (sample HF569108) |

(continued)

| SEQ ID NO | Sequence | Comments |
|---|---|---|
|  | TAAATATAGTAAATTACGGTAAAATATAAATAAGT ACATATTGAAGAAAATGAGACATAATATATTTTAT AATAGGAGGGAATTTCAAATGATAGACAACTTTAT GCAGGTCCTTAAATTAATTAAAGAGAAACGTACC AATAATGTAGTTAAAAAATCTGATTGGGATAAAG GTGATCTATATAAAACTTTAGTCCATGATAAGTTA CCCAAGCAGTTAAAAGTGCATATAAAAGAAGATA AATATTCAGTTGTAGGGAAGGTTGCTACTGGGAA CTATAGTAAAGTTCCTTGGATTTCAATATATGATG AGAATATAACAAAAGAAACAAAGGATGGATATTA TTTGGTATATCTTTTTCATCCGGAAGGAGAAGGC ATATACTTATCTTTGAATCAAGGATGGTCAAAGAT AAGTGATATGTTTCCGCGGGATAAAAATGCTGCA AAACAAAGAGCATTAACTTTATCTTCCGAACTCAA TAAATATATTACATCAAATGAATTTAATACTGGA |  |
| 3 | GCAAAACCTCGCTTTATAGACGAATTCGTTCTAG AGCAATTGAACAGTCATCTTGATAAATTACCCGA ATATATAGCTACGATGACTATGATTGTTCAAGAAT GTGGAATGAGGATAAGTGAATTGTGCACCTTGAA AAAAGGCTGTTTATTAGAGGACAAAGATGGAGAT TACTTTTTAAAGTATTATCAATGGAAAATGAAAAA GGAGCATATAGTTCCAATATCTAAAGAGGTAGTT TTACTTATTAAAGTTCGGGAAGATAAAGTTTCAGA GGAATTTCCAGATAGTGAATACCTCTTTCCAAGA AAAGATGGATCGCCATTAAAACAAGAAACATTTA GAGGCGAATTAAATAAATTAGCTTATGAGCAAAG TATAGTGGATAAATTAGGCGAGATTTATAGATTCC ATGCCCATGCCTTTCGCCATTCAGTAGGAACAAG AATGATTAACAACGGGGTGCCCCAGCATATCGTG CAGAAATTTTTGGGACATGAAAGCCCAGAAATGG CAAGCAGATACGCTCATATCTTTGATCAAACTCTA AAAAATGAATTTACTAAATCTGAAGAAAACTGGT TACCAATAATGGAGATGTG | Second sequence region, wherein the 5' nt is adjacent to the 3' end of the first sequence region. Sequence adjacent to and directly 3' (after) Boundary 49 (sample CP033505) |

(continued)

| SEQ ID NO | Sequence | Comments |
|---|---|---|
| 4 | TCTGTCTTTTTCCTTTTTGACGAAGGCATGAAATA AGTTCGAAATTAATCATATTAGAGTTAATCCATCG ATAGATGGTTTTAAAACAGATTTGGTTTTGTAATA GACGACCTACGATTTGTTCAGGAGACCAGTGGC ATTTTAAATAATATTTAATGATATTACCAAGTTCTG GAGTAAATCGAGTAGGACGACCACAAAGTTTTCT TTTGGTTTCATAATTTTTTTGAGCTGTTTCAGCTT GATATGACTGATTTAAATTATTCCTACTAATTTCA CGTGAGATAGTAGATACAGATCGTTTTAATTTAC GTGCAATAGATCTTAAAGAATAATTTTCTTGACGT AAAACCTCTATACGTGCGCGTTCAGTTAGTGTAA GATGGTTATAGCTCATGTTGGCACTCCTTGTATG TGTTTTTGTGGTTATTAACATCTTACAACAAAGTG CCAATTATGGGCATTTTTTATGAATTTTTATAGGT GTTGCACTTAATATTACAATCCGTCGAGTATAAAA ATGAAATAGATAAAATTAAATCTAAAATTTTAAAT GAAGAAGACGAGTTAGTAAAAAAATCATTGATGT TAACAATATTTGTATTATCGGAGAGCTATGTTAGT TCATTAATTGTTTCTAATTTACCTGAAAGAGATGA AAATTTAATAGATAAAGCGTATGAAAAAATTATTC AACAGTATATAAGTAAAAATATACGTACAAGAAAT GGTAGAAAGAACTAGTAAAAAATTTTTACAATAC AGATCTACCAGCAATACCTCATACCAAATTGAGA GATGCCCTTGCTCACGAAATAAATAAAGTTGATTT AAAAAACAATCGTTTTAAATATGATAATAGTAGAG GTAATAGAAATGAAAATATTATAGAAGTTAGTATT GACGAAATAATTAATGAGTTAGAAGAATGGTTTTT | First sequence region, wherein the 3' nt is adjacent to the 5' end of the second sequence region. Sequence adjacent to and directly before (5') Boundary 8 (sample MFD5994) |
| | AAATTTAGAATTTCCGTAAAAACCTCATCATCAAC CGATAAGCAGAAGCGTATCATAAATGATGCGGTT TTTTCAG | |
| 5 | TGGTCATGCGAAAAACCAAACTTGTTAAACGTTG CAGTGACAAGAGCTAAGAAAGAGTTTTATGTAAT TGGCGACATGCAAAGAATACAGATGAAACCATTT TATGAGACGATTTTTAAAGAAAGAAATGTAAAATG AAATTGAAATAACAGTATTACTTGATACTTAATTAT CGAGCAATTTATACATTTTAAAAGGAGCAAAATTG AATAATGAAGAAATTTAACAACTGGATATTAAATG CAATAAGTGGATCTCAAACAGACAAGAATGAAAC AACTGAAGAATTAAAAGGGGCAAAATTTATCATTT TATATGCATATTCAATGCTCGTTTTGCTTGCGTTA GTAATTTCTAACATATTCATTCACATTTTGGAGCC TAAACTATCAATTACCACTCAAATCATCATCGTTT TGATTTTAATTGAAACACTAATTGGACTGCGTTTC TTGAAAGCGTACGATGTTAAGCGTGGCAAAGATA AAGAAAATAAGAAAAATAGTAAGGATTTCGTTAAA CTAAAATCAATTTTAGTAGCAATTTTATTTACATCA TTGGCGCTGACAGCAGGCACTGTAGCTGATATAT ACGGTTTCACTGACTTAGGAAATACTAGAAGTGA TTTAATCGTTTGGAGCATAGGTGGTATTATATTTG GCCTCGTATGTTACACAATGGAAGATAAAAAATA ACGATAAGGAGCTGGCGATTATAAAGCTAGCTCC TTTTTTAACTTATGTAAATGGCGGTT | First sequence region, wherein the 3' nt is adjacent to the 5' end of the second sequence region. Sequence adjacent to and directly before (5') Boundary 23 (sample HF569108) |

(continued)

| SEQ ID NO | Sequence | Comments |
|---|---|---|
| 6 | AAAAGACCTGATATTGTGTTATTCATAAATGGATT ACCAGTAGTGGTAATTGAACTGAAAAATTCTACA AATGAATCTGTTGGTATTGAAGACGGGTATCATC AGATAGAAACTTATAAAATGAGAACTCCACAATTA TTTAATCATAATGCAGTATTAGTTACGAGTGATGG TATAAATACAAAAGCTGGTTCTTTAACTGCTGAGT ATGATCGTTTTATGACTTGGCGTACTAAAGATGG AAAAACTGAAGATTCATCAACATTTCGTAGTTTAG ATATATTGATTCACGGTATGTTAAATAAAGAGGTA CTATTAGATTTAATTCGTCATTTTGTTTTGTTCCAA GATGATGGTAAAGGAAATATAGTTAAAATATTAG CTGCATATCATCAATATTATGCGGTTAATAAAGCT GTTGATCGTGCTATGGAAGCAACATCTGAACATG GTGATGGTAAGGGCGGCGTTATTTGG | First sequence region, wherein the 3' nt is adjacent to the 5' end of the second sequence region.<br>Sequence adjacent to and directly before (5') Boundary 49 (sample CP033505) |
| 7 | TTCCGTAAAAACCTCATCATCA | Fwd Primer<br>Boundary 8 |
| 8 | CCTACATTTTATAACCGCCATTTAC | Fwd Primer<br>Boundary 23 |
| 9 | CTATGGAAGCAACATCTGAACAT | Fwd Primer<br>Boundary 49 |
| 10 | AAACCTCATATGTTCTGATACATTCAA | Rev Primer<br>Boundary 8 |
| 11 | ACATTCTCTTTAATCCAAACATCAC | Rev Primer<br>Boundary 23 |
| 12 | CATTCCACATTCTTGAACAATCA | Rev Primer<br>Boundary 49 |
| 13 | ACCGATAAGCAGAAGCGTATCATAAA | Probe<br>Boundary 8 |
| 14 | AAGAAAAAGAAAAAAGAAAGCAACAA | Probe<br>Boundary 23 |
| 15 | ACGCCGCCCTTACCATCAC | Probe<br>Boundary 49 |
| 16 | TTCCGTAAAAACCTCATCATCAACCGATAAGCAG AAGCGTATCATAAATGATGCGGTTTTTTCAGCCG<br><br>CTTCATAAAGGGATTTTGAATGTATCAGAACATAT GAGGTTT | Amplicon<br>Boundary 8 |
| 17 | CCTACATTTTATAACCGCCATTTACATGAATCAAA GGTAAAAGAAAAAGAAAAAGAAAGCAACAAAGA GAAGAAGAAATAGAAAAAAGGAGAATAGCAAAAG AAGAGCAAGAAAAGCGTGATGTTTGGATTAAAGA GAATGT | Amplicon Boundary 23 |

(continued)

|  | SEQ ID NO | Sequence | Comments |
|---|---|---|---|
|  | 18 | CTATGGAAGCAACATCTGAACATGGTGATGGTAA GGGCGGCGTTATTTGGGCAAAACCTCGCTTTATA GACGAATTCGTTCTAGAGCAATTGAACAGTCATC TTGATAAATTACCCGAATATATAGCTACGATGACT ATGATTGTTCAAGAATGTGGAATG | Amplicon Boundary 49 |
|  | 19 | AGTACTAGCCATATTAATGCCTCAC | Ü7/10-MRSA-f1 Hiramatsu Boundary fwd primer, in SCCmec |
|  | 20 | CGTATCTATAATAATGAATCGCCTAC | Ü35MRSA-f1 Hiramatsu Boundary fwd primer, in SCCmec |
|  | 21 | CTTAATCAATTTCAAAATIAGTTATATACAACT | Ü36/50-MRSA-f5 Hiramatsu Boundary fwd primer, in SCCmec |
|  | 22 | GCGGCTGAAAAAACCGCATCA | Ü2/5/40/42/43/44MRSA-f2 Hiramatsu Boundary fwd primer, in SCCmec |
|  | 23 | CAATCCTTTTTATATTTAAAATAAATTATACACA | Ü34-f2 Hiramatsu Boundary fwd primer, in SCCmec |
|  | 24 | CTTAATCTTTACTTGTACCTAAATTATCAAAC | SCCmec-R2Ü38-f1 Hiramatsu Boundary fwd primer, in SCCmec |
|  | 25 | ATTATTAATTAATCTGATTTTCACTCTTC | Ü46MRSA-f1 Hiramatsu Boundary fwd primer, in SCCmec |
|  | 26 | CATCTCATTAAATTTTTAAATTATACACA | Ü1/3/45MRSA-f1 Hiramatsu Boundary fwd primer, in SCCmec |
|  | 27 | CTTTATGATAATAGACTTTATAATATAAATACACA | Ü37MRSA-f1 Hiramatsu Boundary fwd primer, in SCCmec |
|  | 28 | CAGGTAGTTTTACCTTCCCACC | Ü41MRSA-f3 Hiramatsu Boundary fwd primer, in SCCmec |
|  | 29 | TAAATCCCTTCCTTTTATATTATICACAA | Ü21/33-MRSA-f1 Hiramatsu Boundary fwd primer, in SCCmec |
|  | 30 | GAGTCACCTCATATTATAIIIIIIACAAC | Ü51MRSA-f2 Hiramatsu Boundary fwd primer, in SCCmec |
|  | 31 | CTCGTAATTTACCTTGTTCATTAAAC | Ü47MRSA-f3 Hiramatsu Boundary fwd primer, in SCCmec |

(continued)

| SEQ ID NO | Sequence | Comments |
|---|---|---|
| 32 | TAAGTATTCAGTATAAAGAATATTTCGCTA | Ü32MRSA-f2 Hiramatsu Boundary fwd primer, in SCCmec |
| 33 | TCCCCATAAAAAAACCTTCCT | Ü52MRSA-f1 Hiramatsu Boundary fwd primer, in SCCmec |
| 34 | AATATATCAAAATTTAGTAATAATTAATATTGCATAC | Ü4/39MRSA-f3 Hiramatsu Boundary fwd primer, in SCCmec |
| 35 | GATCAAACGGCCTGCTCAA | SSC-mec-r12 Hiramatsu Boundary rev primer, binds in orfX |
| 36 | GATCAAATGGCCTGCTCAA | SSC-mec-r13 Hiramatsu Boundary rev primer, binds in orfX |
| 37 | TCAATTAACACAACCCGCATCA | TAQ-SCCmecÜ-13 Hiramatsu Boundary Probe |
| 38 | CAGCAAAATGACATTCCCACATC | TAQ-SCCmecÜ-14 Hiramatsu Boundary Probe |
| 39 | TCATTCAGCAAAATGACATTCCCACATCAAATGATGCGGGTTGTGTTAATTGCACAAGTGTACAGAGCATTTAAGATTATGC | orfX sequence up to Hiramatsu Boundary, SCCmec is adjacent to and after 3' of this sequence (sample MFD5994, also comprising Boundary 8) |
| 40 | GATCAAACGGCCTGCACAAGGACGTCTTACAACGTAGTAACTACGCACTATCATTCAGCAAAATGACATTCCCACATCAAATGATGCGGGTTGTGTTAATTGAGCAAGTGTATAGAGCATTTAAGATTATGC | orfX sequence up to Hiramatsu Boundary, SCCmec is adjacent to and after 3' of this sequence (sample HF569108, also comprising Boundary 23) |
| 41 | GATCAAACGGCCTGCACAAGGACGTCTTACAACGCAGTAACTACGCACTATCATTCAGCAAAATGACATTCCCACATCAAATGATGCGGGTTGTATTAATTGAGCAAGTGTACAGAGCATTTAAGATTATGC | orfX sequence up to Hiramatsu Boundary, SCCmec is adjacent to and after 3' of this sequence (sample CP033505, also comprising Boundary 49) |
| 42 | GTGGAGAGGCGTATCACAAATAAAACTAAAAATGGAGTAACTATTAATATAGTATAAATTCAATATGGTGATAAAAACAGCAAAAAAATAAAATATAACATCATAAAGGTGATGTGAATATATTATACAAAAGGGGAATATAATGTTGGAATTAATTACTGATTTTTCAAAAAAGTCTTTTAAAAATTACAGTATGAAGGAAGAATTAAAATTCAATGCTATAAATATAATATACGGAGTTAATGGGAGGGGAAAAACTTCATTAGCTAGAGGTATTAAAGAAATAATAGAAGAAATAATCCAGATAGCTTGAGGTATTTTTACACTGATTATATTCATGAACTTCTTTTACTAGAAGATTCCAATAAATTTAAAGGGGTAAAAGCGACATTTGGTACAAAAAATGTAGAAATTGAAAATAAAATAATCAAGCTGAAAAATGAAGTTGTTGATATGACGGATACTAAAAAGTTACTAGTTGAGAAGAGAAGGAAGTTAA | SCCmec sequence after Hiramatsu Boundary, orfX (SEQ ID NO 39) is adjacent to and prior to 5' of this sequence (sample MFD5994, also comprising Boundary 8) |

(continued)

| SEQ ID NO | Sequence | Comments |
|---|---|---|
| 43 | GTGGAGAGGCGTATCATAAGTAGGGGTAGTATTA GCCAATTTAATAAATAAAGAGGTGACAGAATGGA CAATCACATAAGAATTTTTGATAATTTATTTCAAA GTAATATTTCAAAATTCCAAAACCTTACTAGTAAA TCATACATTATAAGAAATGATAATGAAAGAATAG TTATCTTCCTATGGTTCAAGAGATAAGAGAGTTGT TTGGAAAGAGGGAGAAATTTTTTCTAAAAGTATA GGAACACATCCTGACTTTTTTGGAATAGAAAATA CTAATGAATACCAGTACATTGTTCCCTGTTTGTA GATATATCTGGGTCTACAAAGCTAGCTTTGAAAT ATAGTTTGGATAAAGTTAAACTTTATAAAAATGCG ATAATTTCTAGTGCTATAGAAATATTTCGTGCATT TGACGGTCATATTCATAGAATACAAGGAGATGCG GTGCTTGTTTATTTTGGACATAAAGAACTGGAAAA AAGTGATGCAATAAT | SCCmec sequence after Hiramatsu Boundary, orfX (SEQ ID NO 40) is adjacent to and prior to 5' of this sequence (sample HF569108, also comprising Boundary 23) |
| 44 | GAGGAGAGGCGTATCATAAGTAGAAGGGGGATT AGCCAATTTAATAAATAAAGGGGTGACAGAATGG ACAATCACATAAAAATTTTGACAATTTATTTCAAA GTAATATTTCAAAATTCCAAAATCTTTCTAGTAAAT CATACATTATTAGAAATGATACTGAAAAGAATAGT TATCTTCCTATGGTTCAAGAGATAAGAGAGTTGTT TGGAAAGAAGGAGAAATTTTTTCTAAAAGTATA GGAACACATCCTGACTTTTTTGAAATAGAAAACA CTAATGAATACCAGTACATTGTTCCCTTTTTGTA GATATATCTGGGTCTACAAAACTAGCTTTGAAATA TAGTTTGGATAAAGTTAAACTTTATAAAAATGCGA TAATTTCTAGTGCTATAGAAATATTTCGTGCATTT GACGGTCATATTCATAGAATACAAGGAGATGCGG TGCTTGTTTATTTTGGACATAAAGAACTGGAAAAA AGTGATGCAATAATT | SCCmec sequence after Hiramatsu Boundary, orfX (SEQ ID NO 41) is adjacent to and prior to 5' of this sequence (sample CP033505, also comprising Boundary 49) |
| 45 | CGATTATAAAGCTAGCTCCTTTTTTAACT | Fwd Primer Boundary 23 |

(continued)

| SEQ ID NO | Sequence | Comments |
|---|---|---|
| 46 | TCTGTCTTTTTCCTTTTTGACGAAGGCATGAAATA AGTTCGAAATTAATCATATTAGAGTTAATCCATCG ATAGATGGTTTTAAAACAGATTTGGTTTTGTAATA GACGACCTACGATTTGTTCAGGAGACCAGTGGC ATTTTAAATAATATTTAATGATATTACCAAGTTCTG GAGTAAATCGAGTAGGACGACCACAAAGTTTTCT TTTGGTTTCATAATTTTTTTGAGCTGTTTCAGCTT GATATGACTGATTTAAATTATTCCTACTAATTTCA CGTGAGATAGTAGATACAGATCGTTTTAATTTAC GTGCAATAGATCTTAAAGAATAATTTTCTTGACGT AAAACCTCTATACGTGCGCGTTCAGTTAGTGTAA GATGGTTATAGCTCATGTTGGCACTCCTTGTATG TGTTTTTGTGGTTATTAACATCTTACAACAAAGTG CCAATTATGGGCATTTTTTATGAATTTTTATAGGT GTTGCACTTAATATTACAATCCGTCGAGTATAAAA ATGAAATAGATAAAATTAAATCTAAAATTTTAAAT GAAGAAGACGAGTTAGTAAAAAAATCATTGATGT TAACAATATTTGTATTATCGGAGAGCTATGTTAGT TCATTAATTGTTTCTAATTTACCTGAAAGAGATGA AAATTTAATAGATAAAGCGTATGAAAAAATTATTC AACAGTATATAAGTAAAAATATACGTACAAGAAAT GGTAGAAAAGAACTAGTAAAAAATTTTTACAATAC AGATCTACCAGCAATACCTCATACCAAATTGAGA GATGCCCTTGCTCACGAAATAAATAAAGTTGATTT AAAAAACAATCGTTTTAAATATGATAATAGTAGAG GTAATAGAAATGAAAATATTATAGAAGTTAGTATT GACGAAATAATTAATGAGTTAGAAGAATGGTTTTT AAATTTAGAAT<u>TTCCGTAAAAACCTCATCATCA</u><u>*ACCGATAA GCAGAAGCGTATCATAAA*</u>TGAT**GCGGTTTTTTCAG**CC GCTTCATAAAGGGATT<u>TTGAATGTATCAGAACATATGA GGTTT</u>ATGTGAATTGCTGTTATGTTTTTAAGAAGCT TATCATAAGTAATGAGGTTCATGATTTTTGACATA GTTAGCCTCCGCAGTCTTTCATTTCAAGTAAATAA TAGCGAAATATTCTTTATACTGAATACTTATAGTG AAGCAAAGTTCTAGCTTTGAGAAAATTCTTTCTGC AACTAAATATAGTAAATTACGGTAAAATATAAATA AGTACATATTGAAGAAAATGAGACATAATATATTT TATAATAGGAGGGAATTTCAAATGATAGACAACTT TATGCAGGTCCTTAAATTAATTAAAGAGAAACGTA CCAATAATGTAGTTAAAAAATCTGATTGGGATAAA GGTGATCTATATAAAACTTTAGTCCATGATAAGTT ACCCAAGCAGTTAAAAGTGCATATAAAAGAAGAT AAATATTCAGTTGTAGGGAAGGTTGCTACTGGGA ACTATAGTAAAGTTCCTTGGATTTCAATATATGAT | Example sequence for MRSA-01 strain (boundary 8 at end of bold, underlined) between sequences associated with CP002388 und CP018768 (see Example 1), comprising Boundary 8 (sample MFD5994) |

(continued)

| SEQ ID NO | Sequence | Comments |
|---|---|---|
| | GAGAATATAACAAAAGAAACAAAGGATGGATATT ATTTGGTATATCTTTTTCATCCGGAAGGAGAAGG CATATACTTATCTTTGAATCAAGGATGGTCAAAGA TAAGTGATATGTTTCCGCGGGATAAAAATGCTGC AAAACAAAGAGCATTAACTTTATCTTCCGAACTCA ATAAATATATTACATCAAATGAATTTAATACTGGA AGATTTTATTACGCAGAAAATAAAGATTCATCTTA TGATTTAAAAAATGATTATCCATCAGGATATTCTC ATGGATCAATAAGATTCAAATATTATGATTTGAAT GAAGGATTCACAGAAGAAGATATGCTAGAGGATT TAAAGAAATTTTTAGAACTATTTAATGAATTAGCTT CAAAAGTTACAAAAACATCCTATGATAGCTTGGT CAATAGCATAGACG | |
| 47 | TCATTCAGCAAAATGACATTCCCACATCAAATGAT GCGGGTTGTGTTAATTGCACAAGT**GTACAGAGC ATTTAAGATTATGC**GTGGAGAGGCGTATCACAAA TAAAACTAAAAATGGAGTAACTATTAATATAGTAT AAATTCAATATGGTGATAAAAACAGCAAAAAAATA AAATATAACATCATAAAGGTGATGTGAATATATTA TACAAAAGGGGAATATAATGTTGGAATTAATTACT GATTTTTCAAAAAAGTCTTTTAAAAATTACAGTAT GAAGGAAGAATTAAAATTCAATGCTATAAATATAA TATACGGAGTTAATGGGAGGGGAAAAACTTCATT AGCTAGAGGTATTAAAGAAATAATAGAAGAAAT AATCCAGATAGCTTGAGGTATTTTTACACTGATTA TATTCATGAACTTCTTTTACTAGAAGATTCCAATA AATTTAAAGGGGTAAAAGCGACATTTGGTACAAA AAATGTAGAAATTGAAAATAAAATAATCAAGCTGA AAAATGAAGTTGTTGATATGACGGATACTAAAAA GTTACTAGTTGAGAAGAGAAGGAAGTTAA | Sequence around the Hiramatsu junction of MRSA-01, the boundary is at the end of the bold marked nucleotides |

(continued)

| SEQ ID NO | Sequence | Comments |
|---|---|---|
| 48 | TGGTCATGCGAAAAACCAAACTTGTTAAACGTTG CAGTGACAAGAGCTAAGAAAGAGTTTTATGTAAT TGGCGACATGCAAAGAATACAGATGAAACCATTT TATGAGACGATTTTTAAAGAAAGAAATGTAAAATG AAATTGAAATAACAGTATTACTTGATACTTAATTAT CGAGCAATTTATACATTTTAAAAGGAGCAAAATTG AATAATGAAGAAATTTAACAACTGGATATTAAATG CAATAAGTGGATCTCAAACAGACAAGAATGAAAC AACTGAAGAATTAAAAGGGGCAAAATTTATCATTT TATATGCATATTCAATGCTCGTTTTGCTTGCGTTA GTAATTTCTAACATATTCATTCACATTTTGGAGCC TAAACTATCAATTACCACTCAAATCATCATCGTTT TGATTTTAATTGAAACACTAATTGGACTGCGTTTC TTGAAAGCGTACGATGTTAAGCGTGGCAAAGATA AAGAAAATAAGAAAAATAGTAAGGATTTCGTTAAA CTAAAATCAATTTTAGTAGCAATTTTATTTACATCA TTGGCGCTGACAGCAGGCACTGTAGCTGATATAT ACGGTTTCACTGACTTAGGAAATACTAGAAGTGA TTTAATCGTTTGGAGCATAGGTGGTATTATATTTG GCCTCGTATGTTACACAATGGAAGATAAAAAATA ACGATAAGGAGCTGG<u>CGATTATAAAGCTAGCTCCTTTTT TAACT</u>**TATGTAAATGGCGGTT**TCATTTCGCCATTTT <u>CCTACATTTTATAACCGCCATTTAC</u>ATGAATCAAAGGTAA *AAGAAAAAGAAAAAGAAAGCAACAA*AGAGAAGAAGAA ATAGAAAAAAGGAGAATAGCAAAAGAAGAGCAAG AAAAGC<u>GTGATGTTTGGATTAAAGAGAATGT</u>ATTTAAAA TTATAGTAAATGAGGAAGGTAAACGAATAATTTTT TGTCCTAAATGTGGTAGTGAGAATAATGTAACTG CTACTACTTGGAACGTAGATGAAGCTGAAAATAG TGAGGATATTGGAACATATTTTAAGAGAACTTGTA | Example sequence of MRSA-02 (HF569108) comprising a boundary (end of bold, underlined) between sequences associated with CP028470 and MRSA-strains (see Example 2), comprising Boundary 23 (sample HF569108). |

(continued)

| SEQ ID NO | Sequence | Comments |
|---|---|---|
| | ATTTATGCGGACATTCTCAAAAATTTAATAAAGAT AACCCTAAAAAAATTTATGAATAGGAATAAAGAAT TGATACATTTTCTGGATAAGTGAAAAAGACAAATT CTATTGAAATAATATAGAAATTGTCTTTAATGTTA GAATTTATAAAACCTCATTATCAACCGATACGCAG AAGCATATCATAAATGATGCGGTTTTTTCAGCCG CTTCATAAAGGGATTTTGAATGTATCAGAACATAT GAGGTTTATGTGAATTGCTGTTATGTTTTTAAGAA GCTTATCATAAGTAATGAGGTTCATGATTTTTGAC ATAGTTAGCCTCCGCAGTCTTTCATTTCAAGTAAA TAATAGCGAAATATTCTTTATACTGAATACTTATA GTGAAGCAAAGTTCTAGCTTTGAGAAAATTCTTTC TGCAACTAAATATAGTAAATTACGGTAAAATATAA ATAAGTACATATTGAAGAAAATGAGACATAATATA TTTTATAATAGGAGGGAATTTCAAATGATAGACAA CTTTATGCAGGTCCTTAAATTAATTAAAGAGAAAC GTACCAATAATGTAGTTAAAAAATCTGATTGGGAT AAAGGTGATCTATATAAAACTTTAGTCCATGATAA GTTACCCAAGCAGTTAAAAGTGCATATAAAAGAA GATAAATATTCAGTTGTAGGGAAGGTTGCTACTG GGAACTATAGTAAAGTTCCTTGGATTTCAATATAT GATGAGAATATAACAAAAGAAACAAAGGATGGAT ATTATTTGGTATATCTTTTTCATCCGGAAGGAGAA GGCATATACTTATCTTTGAATCAAGGATGGTCAA AGATAAGTGATATGTTTCCGCGGGATAAAAATGC TGCAAAACAAAGAGCATTAACTTTATCTTCCGAAC TCAATAAATATATTACATCAAATGAATTTAATACTG GA | |
| 49 | GATCAAACGGCCTGCACAAGGACGTCTTACAAC GTAGTAACTACGCACTATCATTCAGCAAAATGAC ATTCCCACATCAAATGATGCGGGTTGTGTTAATT GAGCAAGT**GTATAGAGCATTTAAGATTATGC**GT GGAGAGGCGTATCATAAGTAGGGGTAGTATTAG CCAATTTAATAAATAAAGAGGTGACAGAATGGAC AATCACATAAGAATTTTTGATAATTTATTTCAAAGT AATATTTCAAAATTCCAAAACCTTACTAGTAAATC ATACATTATAAGAAATGATAATGAAAGAATAGTT ATCTTCCTATGGTTCAAGAGATAAGAGAGTTGTTT GGAAAAGAGGGAGAAATTTTTTCTAAAAGTATAG GAACACATCCTGACTTTTTTGGAATAGAAAATACT AATGAATACCAGTACATTTGTTCCCTGTTTGTAGA TATATCTGGGTCTACAAAGCTAGCTTTGAAATATA GTTTGGATAAAGTTAAACTTTATAAAAATGCGATA ATTTCTAGTGCTATAGAAATATTTCGTGCATTTGA CGGTCATATTCATAGAATACAAGGAGATGCGGTG CTTGTTTATTTTGGACATAAAGAACTGGAAAAAAG TGATGCAATAAT | Sequence of HF569108 (MRSA-02) around the Hiramatsu junction - the junction is at the end of the bold marked nucleotides after 132 base pairs. |

(continued)

| SEQ ID NO | Sequence | Comments |
|---|---|---|
| 50 | AAAAGACCTGATATTGTGTTATTCATAAATGGATT ACCAGTAGTGGTAATTGAACTGAAAAATTCTACA AATGAATCTGTTGGTATTGAAGACGGGTATCATC AGATAGAAACTTATAAAATGAGAACTCCACAATTA TTTAATCATAATGCAGTATTAGTTACGAGTGATGG TATAAATACAAAGCTGGTTCTTTAACTGCTGAGT ATGATCGTTTTATGACTTGGCGTACTAAAGATGG AAAAACTGAAGATTCATCAACATTTCGTAGTTTAG ATATATTGATTCACGGTATGTTAAATAAAGAGGTA CTATTAGATTTAATTCGTCATTTTGTTTTGTTCCAA GATGATGGTAAAGGAAATATAGTTAAAATATTAG CTGCATATCATCAATATTATGCGGTTAATAAAGCT GTTGATCGTG<u>CTATGGAAGCAACA</u>TCTGAACAT<u>G</u>*GTGAT* | Example sequence of MRSA-03 (CP033505/ CP033506) comprising a boundary (end of bold, underlined) between sequences associated with CP020020 and CP032468 (see Example 3) comprising Boundary 49 (sample CP033505). |
| | *GGT***AAGGGCGGCGT**TATTTGGGCAAAACCTCGCTTT ATAGACGAATTCGTTCTAGAGCAATTGAACAGTC ATCTTGATAAATTACCCGAATATATAGCTACGATG ACTA<u>TGATTGTTCAAGAATGTGGAATG</u>AGGATAAGTGA ATTGTGCACCTTGAAAAAAGGCTGTTTATTAGAG GACAAAGATGGAGATTACTTTTTAAAGTATTATCA ATGGAAATGAAAAAGGAGCATATAGTTCCAATA TCTAAAGAGGTAGTTTTACTTATTAAAGTTCGGGA AGATAAAGTTTCAGAGGAATTTCCAGATAGTGAA TACCTCTTTCCAAGAAAAGATGGATCGCCATTAA AACAAGAAACATTTAGAGGCGAATTAAATAAATTA GCTTATGAGCAAAGTATAGTGGATAAATTAGGCG AGATTTATAGATTCCATGCCCATGCCTTTCGCCA TTCAGTAGGAACAAGAATGATTAACAACGGGGTG CCCCAGCATATCGTGCAGAAATTTTTGGGACATG AAAGCCCAGAAATGGCAAGCAGATACGCTCATAT CTTTGATCAAACTCTAAAAAATGAATTTACTAAAT CTGAAGAAAACTGGTTACCAATAATGGAGATGT G | |
| 51 | GATCAAACGGCCTGCACAAGGACGTCTTACAAC GCAGTAACTACGCACTATCATTCAGCAAAATGAC ATTCCCACATCAAATGATGCGGGTTGTATTAATT GAGCAAGT**GTACAGAGCATTTAAGATTATGC**GA GGAGAGGCGTATCATAAGTAGAAGGGGGATTAG CCAATTTAATAAATAAAGGGGTGACAGAATGGAC AATCACATAAAAATTTTGACAATTTATTTCAAAGTA ATATTTCAAAATTCCAAAATCTTTCTAGTAAATCAT ACATTATTAGAAATGATACTGAAAAGAATAGTTAT CTTCCTATGGTTCAAGAGATAAGAGAGTTGTTTG GAAAAGAAGGAGAAATTTTTTCTAAAAGTATAGG AACACATCCTGACTTTTTTGAAATAGAAAACACTA ATGAATACCAGTACATTTGTTCCCTTTTTGTAGAT ATATCTGGGTCTACAAAACTAGCTTTGAAATATAG TTTGGATAAAGTTAAACTTTATAAAAATGCGATAA TTTCTAGTGCTATAGAAATATTTCGTGCATTTGAC GGTCATATTCATAGAATACAAGGAGATGCGGTGC TTGTTTATTTTGGACATAAAGAACTGGAAAAAAGT GATGCAATAATT | Sequence of MRSA-03 (CP033505/ CP033506) around the Hiramatsu junction - the junction is at the end of the bold labelled nucleotides. |

**EXAMPLES**

The invention is further described by the following examples. These are not intended to limit the scope of the invention but represent preferred embodiments of aspects of the invention provided for greater illustration of the invention described herein.

**Example 1:**

The inventors analyzed a patient sample MFD5994 that was confirmed by microbiological cultivation assays to comprise a methicillin-resistant *Staphylococcus aureus* strain (MRSA). Subsequent nucleic acid sequencing of the SA in the sample detected a mecA gene in the sample and thereby confirmed the presence of a MRSA.

Nucleic acid sequencing analysis of the detected SA strain (MRSA-01) revealed the orfX sequence of the MSSA strain CP002388 (the orfX ends in CP002388 at nucleic acid position 34012). Surprisingly, the inventors discovered that the analyzed SA comprised downstream of the orfX cassette sequence of the MSSA strain (starting at sequence position 40039 of the analyzed SA; marked in SEQ ID NO 46 in bold) the sequence of an MRSA strain, such as e.g. CP018768. Sequence analysis showed that the nucleic acid sequence downstream of sequence position 40039 of the analyzed SA was not the one present in CP002388, but resembled the nucleic acid sequence known from, e.g., CP018768 at sequence position 47614 (the orfX of CP018768 ends at 33586 of CP018768). This finding was entirely unexpected, as such a sequence composition has not been reported before in a (MR)SA.

To enable the detection of the newly identified MRSA strain that comprises a sequence of an MSSA strain adjacent to the orfX cassette positioned between said orfX cassette and a MRSA-sequence comprising a mecA gene the inventors designed a set of primers that surround the new junction region disclosed in SEQ ID NO 46 (table 1, bold/underlined) and a probe, which enabled PCR amplification and detection of the newly herein described junction between a MSSA und a MRSA sequence of strain MRSA-001.

The primers and probe sequences used are as follows:

| | |
|---|---|
| Primer forward (SEQ ID NO 7): | TTCCGTAAAAACCTCATCATCA |
| Primer reverse (SEQ ID NO 10): | AAACCTCATATGTTCTGATACATTCAA |
| Probe (SEQ ID NO 13): | ACCGATAAGCAGAAGCGTATCATAAA |

The binding regions of the primers are shown in the underlined nucleotides of SEQ ID NO 46 in table 1. The binding regions of the probe is shown in the underlined italic nucleotides of SEQ ID NO 46 in table 1.

**Example 2:**

The inventors found another MRSA strain HF569108 that comprised the same yet undescribed sequence composition comprising a MSSA-associated sequence in between the orfX cassette and an MRSA sequence that comprises a mecA gene.

Database search of the sequence of this MRSA-02 strain revealed the accession number HF569108. MRSA-02 possesses a nucleic acid sequence known from the MSSA strain CP028470 until sequence position 58456 of CP028470, which comprises the orfX cassette. The orfX cassette of CP028470 ends at sequence position 34026 in MRSA-02.

After the boundary at 58456 of CP028470 (see also SEQ ID NO 48 in table 1, bold/underlined) the sequence does not match CP028470 anymore, but is composed of a short unique sequence of about 48 bases, followed by sequences known from different MRSA strains, such as e.g. CP053101 at 79583. The sequence composition at the boundary region of MRSA-02 is specific for this novel strain and comprises the orfX cassette and sequence of the SCC-mec cassette from the MSSA strain CP028470 (which itself does not comprise a mecA gene), followed by a unique sequence and a sequence region known from different MRSA strains, comprising a mecA gene. To enable the detection of the newly identified MRSA strain the inventors designed a set of primers than surround the new junction region disclosed in SEQ ID NO 48 (table 1, bold/underlined) and a probe, which enabled the PCR amplification and detection of the newly herein described junction between a MSSA und a MRSA sequence of the strain MRSA-002.

The primers and probe sequences used are as follows:

Primer forward 1 (SEQ ID NO 8; located in the unique part of the sequence of MRS-02):

CCTACATTTTATAACCGCCATTTAC

Primer forward 2 (SEQ ID NO 45; upstream of new boundary):

CGATTATAAAGCTAGCTCCTTTTTTAACT

| Primer reverse (SEQ ID NO 11): | ACATTCTCTTTAATCCAAACATCAC |
|---|---|
| Probe (SEQ ID NO 14): | AAGAAAAAGAAAAAAGAAAGCAACAA |

The binding regions of the primers are shown in the underlined nucleotides of SEQ ID NO 48 in table 1. The binding regions of the probe is shown in the underlined italic nucleotides of SEQ ID NO 48 in table 1.

**Example 3:**

The inventors identified a third MRSA strain CP033505 (MRSA-03) that comprised the same yet undescribed sequence composition comprising a MSSA-associated sequence in between the orfX cassette and an MRSA sequence that comprises a mecA gene.

Database search of the sequence of this MRSA-02 strain revealed the accession numbers CP033505 and CP033506. MRSA-03 possesses a nucleic acid sequence known from CP020020, which comprises the orfX (the orfX ends in MRSA-03 at sequence position 33873).

Until sequence position 39478 (location of boundary/junction) the sequence of MRSA-03 resembles the MSSA strain CP020020. After the junction at 39478 of MRSA-03 (bold/underlined in SEQ ID NO 50 in table 1) the sequence is not comprised by CP020020 anymore, but resembles, for example, the MRSA strain CP032468 (at CP032468 sequence position 13616067). This boundary region is specific for MRSA-003.

To enable the detection of the newly identified MRSA strain MRSA-003 the inventors designed a set of primers than surround the new junction region disclosed in SEQ ID NO 50 (table 1, bold/underlined) and a probe, which enabled the PCR amplification and detection of the newly herein described junction between a MSSA und a MRSA sequence of the strain MRSA-03.

The primers and probe sequences used are as follows:

| Primer forward (SEQ ID NO 9): | CTATGGAAGCAACATCTGAACAT |
|---|---|
| Primer reverse (SEQ ID NO 12): | CATTCCACATTCTTGAACAATCA |
| Probe (SEQ ID NO 15): | ACGCCGCCCTTACCATCAC |

The binding regions of the primers are shown in the underlined nucleotides of SEQ ID NO 50 in table 1. The binding regions of the probe is shown in the underlined italic nucleotides of SEQ ID NO 50 in table 1.

PCR methods are carried out and the corresponding amplicons were detected using the methods of the invention.

**Claims**

1. A method to detect a methicillin-resistant *Staphylococcus aureus* (MRSA) strain or genetic material thereof in a sample by a nucleic acid amplification reaction, wherein the MRSA strain comprises a staphylococcal cassette chromosome mec (SCCmec) sequence (16) adjacent to a chromosomal orfX sequence (1),

   wherein the SCCmec sequence (16) comprises:

   - a first sequence region (8) comprising a sequence of a methicillin-sensitive *Staphylococcus aureus* (MSSA), the first sequence (10) positioned adjacent to a chromosomal orfX sequence (1), thereby forming a boundary (9) between said first sequence region (8) and orfX sequence (1), and
   - a second sequence region (10) comprising a sequence of a methicillin-resistant *Staphylococcus aureus* (MRSA) and a mecA gene (17), adjacent to said first region (8), thereby forming a boundary between said first and second regions (11),

   the method comprising:

   contacting a sample with a first primer (12) and a second primer (14), of which at least one hybridizes with the second region (10), capable of generating a first amplicon (15) that comprises a sequence of the second region (10),
   and detecting said first amplicon (15), wherein detecting said amplicon (15) indicates the presence of MRSA or genetic material thereof in the sample.

**2.** The method according to claim 1,
wherein the first primer (12) hybridizes with the first region (8), and the second primer (14) hybridizes with the second region (10), wherein the method comprises generating a first amplicon (15) comprising sequences of the first (8) and second regions (10), and the first amplicon (15) comprises the boundary (11) between said first (8) and second regions (10).

**3.** The method according to claim 1,
wherein the first sequence region (8) is at least 1000 nt in length, preferably 2000 nt, more preferably 3000 nt in length.

**4.** The method according to any of the preceding claims,
comprising additionally:

contacting the sample with a third primer (3) that hybridizes with the orfX sequence (1), and a fourth primer (5) that hybridizes with an adjacent sequence region of an MRSA comprising SCCmec sequence (2), capable of generating a second amplicon (6) comprising SCCmec (2) and orfX (1) sequences and a boundary (7) between said SCCmec (2) and orfX (1) sequences, and
detecting the first and/or second amplicons.

**5.** The method according to the preceding claim,
wherein detecting the first amplicon (15) from the first (12) and second primers (14) of claim 1 or detecting the second amplicon (6) from the third (3) and fourth primers (5) indicates the presence of MRSA or genetic material thereof in the sample.

**6.** The method according to any of the preceding claims,
wherein detecting an amplicon (15, 6) comprises detecting the presence of a signal from an oligonucleotide probe (4, 13) that hybridizes to the sequence of the amplicon (15, 6).

**7.** The method according to any of the preceding claims,
wherein the first (12) and/or second primers (14), and/or the probe (13) for detecting the first amplicon (15), hybridize to a second sequence region (10) that is present in MRSA and absent in MSSA.

**8.** The method according to any of the preceding claims,
wherein the second sequence region (10) comprises a sequence of at least the first 30 nucleotides of a sequence according to SEQ ID NO 1, 2 or 3, or a sequence of at least 80% sequence identity thereto, wherein the first nucleotide of the second sequence region (10) is adjacent to the 3' end of the first sequence region (8).

**9.** The method according to any of the preceding claims,
wherein the first sequence region (8) comprises a sequence of at least the last 30 nucleotides of a sequence according to SEQ ID NO 4, 5 or 6, or a sequence of at least 80% sequence identity thereto, wherein the last nucleotide of the first sequence region (8) is adjacent to the 5' end of the second sequence region (10).

**10.** The method according to any of the preceding claims,
wherein the first (12) and/or second primers (14), and/or the probe (13) for detecting the first amplicon (15) hybridize to a sequence according to SEQ ID NO 1, 2 or 3, or a sequence of at least 80% sequence identity thereto.

**11.** The method according to any of the preceding claims,

wherein the first primer (12) hybridizes with the first region (8) according to SEQ ID NO 4, 5 or 6, or a sequence of at least 80% sequence identity thereto, and
the second primer (14) hybridizes with the second region (10) according to SEQ ID NO 1, 2 or 3, or a sequence of at least 80% sequence identity thereto,
and/or
wherein the first primer (12) comprises or consists of a sequence according to SEQ ID NO 7, 8, 9 or 45, or a sequence of at least 80% sequence identity thereto, and
the second primer (14) comprises or consists of a sequence according to SEQ ID NO 10, 11 or 12, or a sequence of at least 80% sequence identity thereto.

**12.** The method according to any of the preceding claims,

   wherein the probe (13) for detecting the first amplicon (15) from the first (12) and second primers (14) comprises or consists of a sequence according to SEQ ID NO 13, 14 or 15, or a sequence of at least 80% sequence identity thereto, and /or
   wherein the probe for detecting the first amplicon (15) from the first (12) and second primers (14) hybridizes to a sequence according to SEQ ID NO 16, 17 or 18, or a sequence of at least 80% sequence identity thereto.

**13.** The method according to any of the preceding claims,

   wherein the third primer (3) hybridizes with an orfX sequence (1) according to SEQ ID NO SEQ ID NO 39, 40, or 41, or a sequence of at least 80% sequence identity thereto, and the fourth primer (5) hybridizes with an MRSA SCCmec sequence (2), according to SEQ ID NO 42, 43 or 44, or a sequence of at least 80% sequence identity thereto,
   and/or
   wherein the third primer (3) comprises or consists of a sequence according to SEQ ID NO 35 or 36, or a sequence of at least 80% sequence identity thereto, and
   the fourth primer (5) comprises or consists of a sequence according to one of SEQ ID NO 19-34, or a sequence of at least 80% sequence identity thereto.

**14.** The method according to any of the preceding claims,

   wherein the probe for detecting the second amplicon (6) from the third (3) and fourth primers (5) comprises or consists of a sequence according to SEQ ID NO 37 or 38, or a sequence of at least 80% sequence identity thereto, and/or
   wherein the probe for detecting the second amplicon (6) from the third (3) and fourth primers (5) hybridizes to a sequence according to SEQ ID NO 39, 40, or 41, or a sequence of at least 80% sequence identity thereto.

**15.** A kit for detecting a methicillin-resistant *Staphylococcus aureus* (MRSA) strain or genetic material thereof, comprising:

   - a first primer (12) comprising or consisting of a sequence according to SEQ ID NO 7, 8, 9 or 45, or a sequence of at least 80% sequence identity thereto,
   - a second primer (14) comprising or consisting of a sequence according to SEQ ID NO 10, 11 or 12, or a sequence of at least 80% sequence identity thereto,
   - a third primer (3) comprising or consisting of a sequence according to SEQ ID NO 35 or 36, or a sequence of at least 80% sequence identity thereto,
   - a fourth primer (5) comprising or consisting of a sequence according to one of SEQ ID NO 19-34, or a sequence of at least 80% sequence identity thereto,
   - at least one probe (13) for detecting the first amplicon (15) from the first (12) and second primers (14), wherein the probe comprises or consists of a sequence according to SEQ ID NO 13, 14 or 15, or a sequence of at least 80% sequence identity thereto, and/or wherein the probe hybridizes to a sequence according to SEQ ID NO 16, 17, or 18, or a sequence of at least 80% sequence identity thereto, and/or
   - at least one probe (4) for detecting the second amplicon (6) from the third (3) and fourth primers (5), wherein the probe comprises or consists of a sequence according to SEQ ID NO 37 or 38 or a sequence of at least 80% sequence identity thereto, and/or wherein the probe hybridizes to a sequence according to SEQ ID NO 39, 40 and/or 41 or a sequence of at least 80% sequence identity thereto,
   - and optionally means for nucleic acid amplification and/or detection.

Fig. 1

**Fig. 1 continued**

## EUROPEAN SEARCH REPORT

Application Number

EP 23 17 8173

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG MENG ET AL: "Analysis of Staphylococcal Cassette Chromosome mec in BD GeneOhm MRSA Assay-Negative Strains", ANTIMICROBIAL AGENTS AND CHEMOTHERAPY, vol. 57, no. 6, 9 April 2013 (2013-04-09), pages 2890-2891, XP093087814, US ISSN: 0066-4804, DOI: 10.1128/AAC.00174-13 Retrieved from the Internet: URL:https://www.ncbi.nlm.nih.gov/pmc/articles/PMC3716159/pdf/zac2890.pdf> * Fig. 1 A-D; whole document; * | 1-15 | INV. C12Q1/689 |
| X | ZAHNG MENG ET AL: "Supplementary data for XP093087814", ANTIMICROBIAL AGENTS AND CHEMOTHERAPY, vol. 57, no. 6, 9 April 2013 (2013-04-09), pages 1-4, XP093087883, * whole document * | 1-15 | |
| X | ZHANG MENG ET AL: "Supplementary data for XP093087814", ANTIMICROBIAL AGENTS AND CHEMOTHERAPY, vol. 57, no. 6, 9 April 2013 (2013-04-09), pages 1-1, XP093087888, * whole document * | 1-15 | TECHNICAL FIELDS SEARCHED (IPC) C12Q |
| A | WO 2009/018000 A1 (QUEST DIAGNOSTICS INVEST INC [US]; MCCARTHY LARRY [US] ET AL.) 5 February 2009 (2009-02-05) * par. 6 * | 1-15 | |
| A | WO 2009/085221 A2 (BIOMERIEUX SA [US]; JAY CORINNE [FR] ET AL.) 9 July 2009 (2009-07-09) * Fig. 1; par. 2; * | 1-15 | |

The present search report has been drawn up for all claims

| Place of search | Date of completion of the search | Examiner |
|---|---|---|
| Munich | 6 October 2023 | Leber, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 8173

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2023

| Patent document cited in search report | | Publication date | Patent family member(s) | | Publication date |
|---|---|---|---|---|---|
| WO 2009018000 | A1 | 05-02-2009 | EP | 2183391 A1 | 12-05-2010 |
| | | | US | 2009035780 A1 | 05-02-2009 |
| | | | WO | 2009018000 A1 | 05-02-2009 |
| WO 2009085221 | A2 | 09-07-2009 | AU | 2008343878 A1 | 09-07-2009 |
| | | | AU | 2014203649 A1 | 24-07-2014 |
| | | | AU | 2016277561 A1 | 12-01-2017 |
| | | | CA | 2709356 A1 | 09-07-2009 |
| | | | CA | 3046259 A1 | 09-07-2009 |
| | | | CN | 101918593 A | 15-12-2010 |
| | | | EP | 2231869 A2 | 29-09-2010 |
| | | | EP | 2657351 A1 | 30-10-2013 |
| | | | EP | 3434785 A1 | 30-01-2019 |
| | | | ES | 2425267 T3 | 14-10-2013 |
| | | | ES | 2686677 T3 | 19-10-2018 |
| | | | IL | 206499 A | 26-02-2015 |
| | | | JP | 5503552 B2 | 28-05-2014 |
| | | | JP | 6427512 B2 | 21-11-2018 |
| | | | JP | 2011507516 A | 10-03-2011 |
| | | | JP | 2014147388 A | 21-08-2014 |
| | | | JP | 2016152800 A | 25-08-2016 |
| | | | US | 2009203013 A1 | 13-08-2009 |
| | | | US | 2013203056 A1 | 08-08-2013 |
| | | | US | 2016177379 A1 | 23-06-2016 |
| | | | WO | 2009085221 A2 | 09-07-2009 |
| | | | ZA | 201005180 B | 30-03-2011 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6156507 A, Hiramatsu
- WO 2007044873 A2, Huletsky
- WO 2002099034 A
- WO 2009085221 A
- EP 1529847 A
- EP 2807273 A
- WO 2007044873 A

**Non-patent literature cited in the description**

- **WALLACE, R.B. ; SHAFFER, J. ; MURPHY, R.F. ; BONNER, J. ; HIROSE, T. ; ITAKURA, K.** *Nucleic Acids Res.,* 1979, vol. 6, 3543
- **HOWLEY, P.M ; ISRAEL, M.F. ; LAW, M-F. ; MARTIN,M.A.** *J. Biol. Chem.,* vol. 254, 4876
- **FREIER et al.** Improved free-energy parameters for predictions of RNA duplex stability. *Proc Natl Acad Sci USA,* 1986, vol. 83, 9373-9377
- **BRESLAUER, K.J.** Predicting DNA duplex stability from the base sequence. *Proc Natl Acad Sci USA,* 1986, vol. 83, 3746-3750
- **GILL et al.** *Nucleosides Nucleotides Nucleic Acids,* March 2008, vol. 27 (3), 224-43
- **SAMBROOK et al.** Molecular Cloning: A Laboratory Manual. Cold Spring Harbor Laboratory Press, 2001